Europäisches Patentamt

European Patent Office

Office européen des brevets

(19)

(11) **EP 0 613 503 B1**

# (12) FASCICULE DE BREVET EUROPEEN

(45) Date de publication et mention
de la délivrance du brevet:
**12.02.1997 Bulletin 1997/07**

(21) Numéro de dépôt: **93900249.9**

(22) Date de dépôt: **13.11.1992**

(51) Int Cl.$^6$: **C12Q 1/68**

(86) Numéro de dépôt international:
**PCT/FR92/01059**

**WO 93/10257 (27.05.1993 Gazette 1993/13)**

(54) **PROCEDE DE DETERMINATION DE LA QUANTITE D'UN FRAGMENT D'ADN D'INTERET PAR UNE METHODE D'AMPLIFICATION ENZYMATIQUE D'ADN**

VERFAHREN ZUR FESTSTELLUNG DER ANZAHL EINES BESTIMMTEN DNS FRAGMENTS VON INTERESSE BEI DER ENZYMATISCHEN AMPLIFIKATION VON DNS

METHOD FOR DETERMINING THE QUANTITY OF A DNA FRAGMENT OF INTEREST BY ENZYMATIC AMPLIFICATION OF DNA

(84) Etats contractants désignés:
**AT BE CH DE DK ES FR GB GR IE IT LI LU MC NL SE**

(30) Priorité: **15.11.1991 FR 9114089**

(43) Date de publication de la demande:
**07.09.1994 Bulletin 1994/36**

(73) Titulaires:
- **INSTITUT NATIONAL DE LA SANTE ET DE LA RECHERCHE MEDICALE (INSERM)**
**75013 Paris (FR)**
- **INSTITUT PASTEUR**
**F-75015 Paris (FR)**

(72) Inventeurs:
- **PANNETIER, Christophe**
**F-75013 Paris (FR)**
- **COCHET, Madeleine**
**F-92260 Fontenay-aux-Roses (FR)**
- **DARCHE, Sylvie**
**F-91800 Brunoy (FR)**
- **KOURILSKY, Philippe**
**F-75001 Paris (FR)**

(74) Mandataire: **Warcoin, Jacques**
**Cabinet Régimbeau,**
**26, avenue Kléber**
**75116 Paris (FR)**

(56) Documents cités:
EP-A- 0 461 496          WO-A-91/02817
WO-A-92/01812

- **NUCLEIC ACIDS RESEARCH, vol. 17, no. 22, 1989, Arlington, VA (US); M. BECKER-ANDRE et al., pp. 9437-9446**
- **PROCEEDINGS OF THE NATL. ACADEMY OF SCIENCES OF USA, vol. 87, Avril 1990, Washington, DC (US); G. GILLILAND et al., pp. 2725-2729/**

EP 0 613 503 B1

**Description**

La présente invention se rapporte aux méthodes d'amplification enzymatique in vitro de séquences d'acides nucléiques ADN mono-brins ou double brins, notamment par PCR (Polymerase Chain Reaction) ou par la technique par SDA (Strand Displacement Amplification). Plus précisément, la présente invention concerne un perfectionnement de ces méthodes d'amplification permettant de quantifier, après amplification, la séquence d'intérêt initiale.

Les techniques d'amplification enzymatique d'ADN sont bien connues de l'homme de l'Art.

Les techniques d'amplification in vitro, notamment par PCR ou SDA, ont été décrites dans la littérature et leur mise en oeuvre et perfectionnements ont fait l'objet de demandes de brevet. On peut citer en particulier les publications européennes EP 201 184 et EP 200 362 sur la technique de base pour la méthode PCR. La technique d'amplification dite par SDA (Strand Displacement Amplification) a été décrite lors de la conférence de San Diego sur les Acides Nucléiques les 20-22 novembre 1991.

Dans ces méthodes d'amplification enzymatique d'ADN, l'amplification de la séquence s'effectue par cycles successifs. Un cycle comporte plusieurs étapes : une première étape de dénaturation thermique de l'ADN qui sépare, le cas échéant, l'ADN double brin en deux mono-brins, une seconde étape d'hybridation des amorces sur les extrémités 5' des séquences des mono-brins qui leur sont complémentaires, et une troisième étape d'élongation à partir des extrémités 3' des amorces à l'aide d'une ADN polymérase. On utilise des amorces oligonucléotides spécifiques qui vont s'hybrider sur des séquences qui leur sont complémentaires encadrant à ses extrémités 5'P le fragment d'ADN à amplifier. Les extrémités 3'OH de ces amorces constituent le point de démarrage d'une élongation effectuée, dans le sens 5'→ 3', par les enzymes de replication de l'ADN appelées ADN polymérases.

Pour une amplification, le milieu réactionnel contient donc l'ADN à amplifier, les amorces, l'ADN polymérase, ainsi que d'autres éléments tels qu'un tampon, des sels, des désoxynucléotides tri-phosphates. Chaque brin du fragment sert alors de matrice à l'enzyme qui synthétise les brins complémentaires ; le facteur de multiplication est alors de deux. Les oligonucléotides "amorces" (primer) sont à nouveau remis à hybrider avec les brins d'ADN provenant du premier cycle d'amplification, chaque brin servant de matrice à l'ADN polymérase. Le facteur d'amplification sera alors de quatre - et ainsi de suite - avec doublement théorique des copies du fragment d'ADN à chaque cycle.

Dans certaines techniques d'amplification enzymatique d'ADN telles que la technique PCR, les produits issus de ce premier cycle, puis de chacun des cycles successifs, sont dénaturés par la chaleur. L'emploi d'une polymérase thermostable, la Taq polymérase, a permis de développer des cycleurs automatiques (hybridation/polymérisation enzymatique/dénaturation thermique), rendant plus aisé l'emploi routinier de la méthode. Les conditions opératoires des différentes étapes se distinguent en effet essentiellement par la température à laquelle elles ont lieu. Les conditions de dénaturation correspondent généralement à une élévation de la température du milieu réactionnel au-dessus de 90°C, l'hybridation a lieu généralement entre 50 et 70°C et l'élongation par l'ADN polymérase peut s'effectuer à des températures relativement élevées, de l'ordre de 70°C si l'on utilise une ADN polymérase stable à la chaleur.

En revanche, dans d'autres techniques d'amplification enzymatique d'ADN, telles que la technique par SDA, les produits issus du premier cycle comme de chacun des cycles successifs ne sont pas dénaturés. Il s'agit de méthode isothermes.

La méthode d'amplification par SDA repose sur l'utilisation d'amorces oligonucléotidiques modifiées en 5' par l'addition d'une séquence d'ADN reconnue par une enzyme de restriction, par exemple l'enzyme Hinc II. Le processus requiert la formation d'un site de restriction thiole par incorporation de déoxyadénosine triphosphate soufré (ci-après d'ATPs), et les actions alternées de ladite enzyme Hinc II qui hydrolyse partiellement (sur un seul brin), ledit site de restriction, et de l'ADN Polymérase qui synthétise à partir du point d'hydrolyse un nouveau brin, avec déplacement simultané de la séquence nucléique précédemment coupée, sans que la dénaturation soit nécessaire. L'hybridation des amorces modifiées sur la cible nécessite seulement une première étape de dénaturation de l'ADN. La réaction se fait ensuite à 37°C. La méthode permet une amplification moyenne de la cible de l'ordre de $10^7$.

Le milieu comprend les réactifs suivants :

+ d TTP, dGTP, dCTP et dATPs
+ ADN Polymérase
+ ADN Cible à amplifier
+ L'enzyme Hinc II. une séquence reconnue par l'enzyme Hinc II est :

$$\text{GTTGAC}$$
$$\text{CAACTG}$$

La flèche indique le site de clivage.

L'amorce modifiée en 5' par couplage à la séquence GTTGAC est reconnue par l'enzyme Hinc II.

A partir d'un des monobrins résultant de la dénaturation inititale de l'ADN à amplifier, un schéma d'amplification illustratif est le suivant :

(1) Hybridation de l'amorce sur le brin complémentaire :

G T T G A C_____
                    ‖‖‖‖‖
                        _____ADN cible monobrin

(2) Synthèse du brin complémentaire par la DNA Polymérase :

L'incorporation de dATPs conduit à la formation d'un site de restriction thiolé qui est incomplètement reconnu par Hinc II et n'est donc pas clivé

G T T G A C _____

C AsAsC T G _____

(3) Hydrolyse partielle du site de restriction par Hinc II :

La coupure n'a lieu que sur le brin non thiolé.

G T T↓G A C _____

C AsAsC T G _____

(4) Synthèse du brin d'ADN par l'ADN Polymérase : La polymérase "s'ancre" au niveau du site hydrolysé (4-1).

(4-1) :

G A C____
G T T G AsC ───▶
C AsAsC T G _____  + DNA Polymérase

Elle resynthétise un brin correspondant au brin coupé, en déplaçant ce dernier au fur et à mesure que la synthèse s'effectue (4-2).

(4-2) :

G A C _____

G T T↓G AsC _____

C AsAsC T G _____

(5) Nouvelles actions de Hinc II et de l'ADN polymérase, conduisant à de nouvelles synthèses avec déplacement des brins hydrolysés.

```
G  A  C  _____

G  AsC  _____
                                \
G  T  T  G  AsC  _____→  _____

C  AsAsC  T  G  _____


G  A  C  _____

G  AsC  _____

G  AsC  _____

G  AsC  _____

G  T  T↓G  AsC  _____

CAsAs  C  T  G  _____
```

La réaction se fait simultanément sur les deux brins d'ADN cible, ce qui conduit à une augmentation exponentielle du nombre de copies en fonction du temps de réaction.

Une amplification par un facteur $10^7$ peut être obtenue en 2 heures à 37°C.

Dans toutes les méthodes d'amplification, théoriquement, après un premier cycle, une séquence d'intérêt à amplifier présente en $y_o$ exemplaires dans l'échantillon à tester se trouvera dupliquée, en 2 $y_o$ exemplaires. Après n cycles d'amplification, le nombre de séquences cibles sera multiplié par $2^n$, c'est-à-dire qu'une séquence cible présente initialement en $y_o$ exemplaires sera présente en $y = y_o \cdot 2^n$ exemplaires. Toutefois, les efficacités expérimentales ne sont pas de 100% à chaque cycle. L'efficacité ou rendement de la réaction "eff" au cours d'un cycle n'est pas de 100%, on obtient plutôt la valeur du nombre de séquences par : $y = Y_o \cdot (1 + eff)^n$ plutôt que $x2^n$ avec eff < 1.

Une des difficultés des méthodes d'amplification enzymatique d'ADN est en effet d'obtenir une réaction quantitative, c'est-à-dire de pouvoir quantifier précisément la quantité d'ADN dans un échantillon, ou les valeurs relatives de deux fragments d'ADN dans un échantillon donné.

Pour surmonter ce problème, plusieurs méthodes ont été envisagées (Wang et al., 1989 , Gilliland, 1990). Elles partagent toutes en commun les caractéristiques suivantes :

a) On effectue un étalonnage interne à l'aide d'échantillons d'acides nucléiques standards de concentrations connues, amplifiables par les mêmes oligonucléotides amorces que ceux utilisés pour amplifier les séquences d'intérêts, et

b) on réalise une co-amplification des séquences d'intérêt et standards et on stoppe les réactions d'amplification à intervalles de deux ou trois cycles, après un nombre relativement réduit de cycles, c'est-à-dire pendant que l'amplification est en phase exponentielle et avant la saturation de l'amplification ou en phase stationnaire, puis

c) on sépare les produits d'amplification entre séquences standard d'ADN et séquences d'ADN d'intérêt amplifiées, selon leur taille, par des techniques connues, telles que l'électrophorèse sur gel et on peut déterminer, par des moyens connus, la quantité de séquence d'ADN d'intérêt initiale, à partir de la quantité initiale de séquences standards et du rapport des quantités de séquences standards amplifiées et séquences d'intérêts amplifiées, en considérant que le rapport des quantités des séquences initiales et amplifiées est identique.

Dans une technique antérieure (Singer-Sam et al., 1990 et Robinson and Simon, 1991) , la méthode consistait, en variant le nombre de cycles d'amplification, à mesurer les rendements (Y) tous les deux ou trois cycles, pendant que la réaction d'amplification s'effectue dans sa phase exponentielle. Le rendement (Y) obéit alors à l'équation $Y = Y_o(1 + eff)^n$ où n est le nombre de cycles et eff l'efficacité de la réaction dans un cycle.

Cependant on a découvert qu'il n'est pas possible d'effectuer un étalonnage préalable avant l'analyse à effectuer mais qu'il est nécessaire d'avoir recours à un étalon interne au cours de l'analyse et de l'amplification, si l'on veut une quantification précise, et ce vraisemblablement en raison du très grand nombre de paramètres qui interviennent au cours de l'amplification et de leurs caractères très variables qui influent sur le rendement de l'amplification et donc sa quantification.

Dans les méthodes d'amplification enzymatique d'ADN, au bout d'un certain nombre de cycles, il se produit une phase de saturation ou phase stationnaire avec apparition d'un plateau, les produits spécifiques d'amplification (séquences d'intérêt et standard) n'étant plus amplifiés. En effet, à ce moment, pourvu que l'enzyme ADN-polymérase soit en quantité suffisante, les produits d'amplification sont en grand nombre et rentrent en compétition avec les amorces qui, elles, sont en quantité moins importantes, et déplacent l'équilibre vers une hybridation des produits spécifiques d'amplification entre eux, plutôt qu'avec les amorces.

Le nombre de cycles au bout duquel cette phase de saturation apparaît est variable en fonction de la quantité initiale du produit à amplifier et des quantités et de l'efficacité des réactifs utilisés (amorces et polymérases en particulier) dans la réaction d'amplification.

Toutefois, dans des conditions usuelles de réalisation de l'amplification, ce nombre de cycles sera d'environ 40.

Dans la technique antérieure mentionnée ci-dessus, on évite impérativement d'effectuer les mesures de quantification une fois la saturation atteinte ou dans les derniers cycles avant saturation. En effet, cette saturation de l'amplification des séquences spécifiques entraîne une augmentation du bruit de fond dû à l'amplification de produits non spécifiques qui, eux, continuent à s'amplifier exponentiellement. Ces produits d'amplification non spécifiques correspondent à des séquences d'ADN présentant une homologie de séquence avec la séquence d'ADN d'intérêt de sorte qu'elles sont amplifiées par les mêmes amorces ; leur amplification commence à se produire essentiellement dans les derniers cycles avant la phase stationnaire.

Il était donc nécessaire, avant l'invention, de faire plusieurs mesures à intervalles réguliers, tous les deux ou trois cycles de l'amplification, comme on l'a mentionné, pour vérifier que la mesure prise en compte a bien été effectuée à un stade suffisamment avancé mais, toutefois, suffisamment tôt avant la saturation, dans la mesure où l'amplification des séquences non spécifiques démarre quelques cycles avant la phase stationnaire. Ceci est une contrainte pratique très importante.

Un autre problème pratique majeur de cette technique antérieure est que l'on oberve des variations significatives de l'amplification de tube à tube, pendant la phase exponentielle, en particulier parce que le rendement des amorces est, pendant cette phase, très variable. Ceci réduit les précisions des mesures et les rend échantillon-dépendantes. Il s'agit d'un inconvénient important si l'on veut effectuer et comparer, comme c'est le cas dans la pratique, plusieurs analyses en parallèle sur des échantillons d'origines diverses.

Dans la technique antérieure, la quantification relative des fragments d'ADN d'intérêt et standards amplifiés peut se faire :

- soit en utilisant une amorce marquée au cours de l'amplification,

  - Dans ce cas, les fragments d'ADN d'intérêt et standards sont séparés selon leur taille par électrophorèse sur gel et l'intensité du signal correspondant au marqueur permet ensuite de quantifier relativement les fragments d'ADN d'intérêt et standards amplifiés.

- soit, après amplification, par hybridation sur gel avec des sondes ADN marquées, permettant de distinguer les fragments d'ADN d'intérêt et standards.

Le but de la présente invention est de fournir une méthode de quantification d'un fragment d'ADN par une méthode d'amplification enzymatique plus facile à mettre en oeuvre, plus précise, plus fiable et reproductible d'échantillon à échantillon.

Pour ce faire, la présente invention a pour objet un procédé de détermination de la quantité d'un fragment d'ADN d'intérêt d'un échantillon à analyser par une méthode d'amplification enzymatique in vitro dudit fragment, caractérisé en ce que:

1) on ajoute à l'échantillon à analyser contenant un dit fragment d'ADN d'intérêt, une quantité déterminée d'un fragment d'ADN standard différent du dit fragment d'ADN d'intérêt mais amplifiable par les mêmes oligonucléotides amorces, les fragments d'ADN standards et d'intérêt étant aussi proches que possible, notamment ne différant en séquence et/ou en taille de pas plus de 10% de nucléotides, de préférence de pas plus de 5 nucléotides par brin,
2) on co-amplifie les fragments d'ADN intérêt et standards avec les mêmes oligonucléotides amorces, de préférence jusqu'à saturation de l'amplification du fragment d'ADN d'intérêt ou dans les derniers cycles précédant la saturation,
3) on ajoute dans le milieu réactionnel obtenu à l'étape 2) :

  - soit deux types d'oligonucléotides marqués sondes spécifiques chacun respectivement des fragments d'ADN d'intérêt et standards, et différents des oligonucléotides amorces d'amplification de l'étape 2),
  - soit un ou plusieurs oligonucléotide(s) amorce(s) marqué(s). spécifique(s) des fragments d'ADN d'intérêt et standards, et différents des oligonucléotides amorces d'amplification de l'étape 2), et on effectue un ou quelques cycle(s) d'amplification supplémentaire(s) avec la ou lesdites amorce(s) avec une ADN polymérase, de sorte que, au cours d'un cycle, après dénaturation de l'ADN, le ou lesdits oligonucléotide(s) amorce(s) marqué (s) s'hybride(nt) avec lesdits fragments en un site approprié pour qu'une élongation par l'ADN polymérase génère des fragments d'ADN marqués de tailles et/ou de séquences différentes et/ou avec des marqueurs différents selon qu'ils proviennent des fragments d'ADN d'intérêt ou standards respectivement, puis

4) on détermine la quantité initiale de fragment d'ADN d'intérêt comme étant le produit de la quantité initiale de fragment d'ADN standard et du rapport entre la quantité de fragment d'ADN d'intérêt amplifié et la quantité de fragment d'ADN standard amplifié, rapport qui est identique à celui des quantités des fragments d'ADN marqués provenant respectivement des fragments d'ADN d'intérêt et standards éventuellement amplifiés obtenus à l'étape 3).

A l'étape 3, on entend par "sonde" une séquence d'ADN qui s'hybride spécifiquement avec respectivement l'ADN d'intérêt ou standard.

A l'étape 3, on entend par "amorce(s)" une ou des séquence(s) d'ADN qui s'hybrident spécifiquement en un ou des site(s) approprié(s) pour qu'une élongation subsequente par l'ADN polymérase génère des fragments d'ADN marqués de tailles et/ou de séquences différentes et/ou avec des marqueurs différents selon qu'ils proviennent de fragments d'ADN d'intérêt ou standard.

A l'étape 4, lorsque les oligonucléotides marqués dans l'étape 3 sont des sondes d'ADN s'hybridant spécifiquement avec respectivemnet l'ADN d'intérêt et l'ADN standard, on entend par "fragments d'ADN marqués provenant respectivement de fragments d'ADN d'intérêt et standards", les duplex résultant de l'hybridation des sondes sur les fragments d'ADN d'intérêt et standards respectivement.

La détection et la quantification des fragments d'ADN d'intérêt et standards se font alors selon les méthodes conventionnelles des sondes d'ADN.

L'hybridation ou l'élongation spécifique des matrices constituées des produits d'amplification (ADN standard et d'intérêt ) à l'étape 3), à l'aide respectivement de sondes ou d'amorces qui ne s'hybrident que sur ces derniers ou respectivement ne permettent de recopier partiellement que ces derniers, permet de les distinguer des produits d'amplification non spécifiques, bien que ceux-ci soient en quantité importante à la phase de saturation, dans la mesure où seuls les produits spécifiques sont désormais marqués.

Un autre avantage de la méthode selon la présente invention provient de ce qu'on observe qu'une fois la saturation de l'amplification atteinte ou en fin de phase exponentielle, il n'y a plus de différence de rendement des amorces, et partant, de l'amplification, d'échantillon à échantillon, c'est-à-dire de tube à tube, différence qui apparaissait en phase d'amplification exponentielle dans la technique antérieure. Au plateau, une uniformisation est atteinte, ce qui rend la mesure selon le procédé de l'invention précise, fiable et reproductible de tube à tube, pour cette raison supplémentaire.

Un autre avantage de la méthode selon l'invention résulte de sa plus grande sensibilité compte tenu que les produits sont amplifiés en plus grand nombre à saturation.

A l'étape 3), on effectuera le cas échéant de préférence un seul cycle d'amplification supplémentaire ou, en tous les cas, un nombre assez réduit par exemple pas plus de 5, de manière à éviter l'apparition d'un nouveau bruit de fond.

On notera que les fragments d'ADN standards et d'intérêt ont nécessairement leurs extrémités identiques puisqu'ils sont amplifiés par les mêmes amorces au cours de l'amplification de l'étape 2. D'autre part, on notera également que les fragments d'ADN standards et d'intérêt sont de même nature, à savoir monobrins ou doubles brins.

A l'étape 3), lorsqu'on utilise deux types d'oligonucléotides marqués en tant que sondes spécifiques respectivement de l'ADN d'intérêt et de l'ADN standard, il n'est pas nécessaire d'effectuer un ou quelques cycles d'amplification supplémentaires. Le marqueur des deux sondes peut, dans ce cas, être identique, auquel cas il est nécessaire de fractionner au préalable l'échantillon contenant les produits amplifiés, obtenu à l'étape 2), en deux fractions et d'ajouter dans chaque fraction une sonde de séquence d'ADN différente. Ou le marqueur peut être différent, auquel cas il est possible de déterminer la quantité respectivement des fragments d'ADN d'intérêt et standard dans le même échantillon en détectant directement l'intensité du signal correspondant à chacun des marqueurs.

Selon l'invention, les fragments d'ADN d'intérêt et standards ont une longueur et une séquence aussi proches que possible. De préférence, le fragment standard porte une modification aussi mineure que possible par rapport à l'ADN d'intérêt et diffère en au moins un site repérable par quelque moyen que ce soit. Ceci garantit une plus grande similitude de leur taux d' amplification, ce qui est un élément capital pour la précision et la fiabilité de la méthode pour la reproduction d' échantillon à échantillon.

Dans la présente demande, on entend par l'expression "différent en séquence" qu'un certain nombre de nucléotides entrant dans la constitution des fragments d'ADN standard et ADN à quantifier, sont différents.

Les fragments d'ADN à quantifier et ADN standard peuvent avoir la même taille, auquel cas l'un des fragments devra comporter un "site repérable", c'est-à-dire permettant de le distinguer de l'autre fragment par quelques moyens notamment par coupure spécifique du fragment par un réactif particulier tel qu'une enzyme de restriction.

Toutefois en général, cette différence de séquence s'accompagne d'une différence de taille.

De préférence, les tailles des fragments d'ADN d'intérêt et standards diffèrent d'au moins 1 nucléotide et de pas plus de 10% environ de nucléotides par brin ou de 1 à 5 nucléotides.

En particulier, on a découvert selon l'invention que quel que soit le réactif ou le paramètre qui limite la réaction d'amplification, l'amplification des deux molécules (fragment d'ADN d'intérêt et fragment d'ADN standard) évolue alors de la même manière et ce même à saturation, de sorte que le rapport des quantités respectives des fragments d'ADN d'intérêt et standards amplifié reste identique à celui des molécules de départ, ce qui n'est pas le cas lorsque les séquences de ces deux molécules diffèrent de façon plus importante.

Lorsque les séquences d'ADN d'intérêt ont entre 50 et 500 paires de bases (pour les séquences doubles brins) ou nucléotides (pour les séquences monobrins), les amorces ont environ 15 à 25 nucléotides, de sorte que, de préférence, la différence de taille et/ou séquence entre fragments d'ADN standards et fragments d'ADN d'intérêt ne dépasse pas 10%.

Toutefois, de façon appropriée, lorsque la taille du fragment d'ADN d'intérêt est estimée entre 50 et 500 paires de bases ou nucléotides, la différence de taille et/ou séquence des fragments d'ADN standards et d'intérêt sera de deux à cinq nucléotides.

Pour les séquences d'ADN d'intérêt de moins de 50 paires de base, les différences en séquences et/ou tailles des fragments d'ADN d'intérêt et standards sont comprises entre un et cinq.

D'une manière appropriée, la différence de séquence du fragment d'ADN standard pourra résulter d'une ou plusieurs délétion(s), mutation(s) ou addition(s) de nucléotides dans la séquence d'ADN d'intérêt réalisée par les techniques de génie génétique conventionnelles, les séquences d'ADN à quantifier et standards étant par ailleurs identiques.

Dans un mode de réalisation particulier de l'invention, à l'étape 3), on ajoute dans le milieu réactionnel obtenu à l'étape 2) un troisième oligonucléotide amorce marqué, amorce commune aux fragments d'ADN d'intérêt et standards, et on effectue un ou plusieurs cycle(s) d'amplification supplémentaire(s) avec ladite troisième amorce, tel que, après dénaturation de l'ADN, ladite troisième amorce s'hybride spécifiquement avec lesdits fragments en un site approprié pour qu'une élongation par l'ADN polymérase génère deux types de fragments d'ADN marqués de tailles différentes selon qu'ils proviennent des fragments d'ADN d'intérêt et standards respectivement et comportant chacun une partie seulement de ces derniers respectivement.

Le plus généralement, le fragment d'ADN standard différera de l'ADN d'intérêt en un seul site où l'on aura opéré une mutation, délétion ou addition par les techniques connues de génie génétique du nombre de nucléotides choisi, en particulier de 1 à 5, de préférence 3 à 4 nucléotides. Dans ce cas, ladite troisième amorce s'hybride en amont de ce site où les deux séquences divergent de sorte que l'élongation se faisant d'amont en aval (5' → 3') les séquences d'ADN marquées après élongation conservent une différence de taille selon qu'elles proviennent du fragment d'ADN d'intérêt ou du fragment d'ADN standard.

Dans un autre mode de réalisation, les modifications qui permettent de distinguer l'ADN standard et l'ADN à quantifier ne portent plus sur la taille mais sur la présence d'une modification détectable telle qu'un site de restriction dans l'un d'eux.

La présente invention a donc également pour objet un procédé de détermination de la quantité d'un ADN d'intérêt caractérisé en ce que:

a) les fragments d'ADN standards et d'intérêt ont la même taille et diffèrent en séquence par un site de restriction d'une endonucléase particulière, ou site de coupure réalisable par tout autre moyen, et

b) à l'étape 3), on ajoute au milieu réactionnel obtenu à l'étape 2) un troisième oligonucléotide amorce marqué, amorce commune aux fragments d'ADN d'intérêt et standards, et on effectue un ou quelques cycle(s) d'amplification supplémentaire(s) avec ledit troisième oligonucléotide amorce, de sorte qu'au cours d'un cycle, après dénaturation de l'ADN, ladite troisième amorce s'hybride spécifiquement avec lesdits fragments en un site approprié pour qu'une élongation par l'ADN polymérase génère deux types de fragments d'ADN marqués se distinguant selon qu'ils proviennent des fragments d'ADN d'intérêt et standards respectivement, en ce que l'un desdits fragments comporte toujours ledit site de restriction et

c) avant l'étape 4), on effectue une digestion des produits d'amplification de l'étape 3), à l'aide de ladite endonucléase ou tout autre moyen de coupure correspondant au site de coupure, de manière à générer des fragments de tailles différentes selon qu'ils proviennent des fragments d'ADN d'intérêt ou standards.

L'introduction ou l'élimination d'un site de restriction d'endonucléase peut se faire par mutagenèse dirigée ou tout autre méthode connue.

Dans un premier mode de réalisation, à l'étape 4) du procédé selon l'invention, la mesure du rapport des quantités d'ADN standards et d'ADN à quantifier requiert préalablement la séparation physique de ces deux espèces, cette séparation pouvant se faire par électrophorèse sur gel, ou d'autres méthodes connues de l'homme de l'art.

Ainsi, de façon appropriée, la détermination à la dernière étape selon le procédé de l'invention se fait en :

- séparant selon leur taille par électrophorèse sur gel les fragments d'ADN marqués provenant des fragments d'ADN d'intérêt et standards amplifiés, puis en
- détectant les intensités des signaux correspondant au marqueur de ladite troisième amorce respectives pour les fragments d'ADN marqués provenant des fragments d'ADN d'intérêt et standards respectivement.

On utilisera avantageusement des "gels séquenceurs" consistant en des dispositifs d'électrophorèse sur gel avec séquençage d'ADN qui permettent de distinguer et séparer des séquences différant de un nucléotide seulement.

Comme marqueur, on peut avoir recours de façon classique notamment à un marquage radioactif, enzymatique ou fluorescent, selon les techniques bien connues de l'homme de l'art.

Toutefois, on utilisera tout particulièrement un marqueur fluorescent, ce qui permet d'utiliser de façon tout à fait appropriée un dispositif automatisé d'électrophorèse sur gel à séquençage d'ADN automatique, décrit dans US 4 811 218 et correspondant en particulier au dispositif commercialisé par Applied Biosystem, modèle 373A$^R$. Avec ce dispositif, un logiciel approprié permet de numériser la surface de pics de fluorescence enregistrés pour chacun des fragments d'ADN de tailles diverses et rendre compte de leurs quantités relatives.

La synthèse d'oligonucléotides marqués avec un marqueur fluorescent est bien connue de l'homme de l'art.

L'appareil est donc utilisé uniquement pour mesurer les longueurs et l'intensité des produits d'élongation, Bien entendu, puisqu'il existe quatre fluophores commerciaux de "couleurs" différentes, on pourra, comme il est fait dans les déterminations de séquence d'ADN, mais pour des raisons différentes utiliser des fluorophores différents.

Certains appareils commerciaux pratiquent une analyse des quatre couleurs dans 24 pistes simultanément (soit 96 échantillons). L'analyse prend quelques heures et les résultats sont informatisés.

En outre, la méthode selon l'invention offre la possibilité de réaliser des analyses multiparamétriques permettant de doser et quantifier plusieurs fragments d'ADN de tailles différentes dans un même échantillon analysé, pourvu que les fragments d'ADN marqués provenant des ADN standards et à quantifier aient des tailles différentes entre eux respectivement, d'une part, et fournissent des fragments d'ADN échelonnés en tailles détectables en une seule détermination de taille, c'est-à-dire sur une seule piste de gel de séquence. Pour ce faire, il suffit de faire un choix approprié des oligonucléotides amorces d'amplification ou marqués, de manière à générer, au cours de l'élongation de l'étape 3, des fragments de tailles différentes.

On peut alors, en une seule détermination de taille, doser et quantifier jusqu'à 50 gènes dans un même échantillon.

La présente invention a également pour objet un procédé selon l'une des revendications précédentes d'analyse multiparamétrique permettant de doser et quantifier plusieurs fragments d'ADN d'intérêt dans un même échantillon analysé, caractérisé en ce que :

- on ajoute à l'échantillon à analyser, contenant lesdits fragments d'ADN d'intérêt, autant de fragments d'ADN standards, les fragments d'ADN standard étant différents pour chacun des fragments d'ADN d'intérêt, et
- on choisit les ADN standards et les oligonucléotides amorces marqués de l'étape 3), de façon à générer, par élongation, au cours de l'étape 3), des fragments de tailles différentes.

Dans d'autres modes de réalisation, la présente invention a pour objet un procédé caractérisé en ce que, à l'étape 3), on ajoute dans du milieu réactionnel obtenu à l'étape 2) deux oligonucléotides amorces différents des amorces d'amplification de l'étape 2), spécifiques des fragments d'ADN d'intérêt et standards respectivement et marqués par des marqueurs différents; puis on effectue, le cas échéant, un ou plusieurs cycle(s) d'amplification supplémentaire(s) avec lesdits deux oligonucléotides amorces marqués, de sorte qu'au cours d'un cycle, après dénaturation de l'ADN et élongation par une ADN polymérase, les deux dits oligonucléotides marqués génèrent deux types de fragments d'ADN marqués par des marqueurs différents selon qu'ils proviennent d'ADN d'intérêt ou standards.

Dans ces cas, à l'étape 4), la séparation physique de l'ADN standard et de l'ADN à quantifier est facultative si l'on détecte directement l'intensité du signal correspondant à chacun des marqueurs.

En effet, la mesure du rapport des quantités d'ADN standard et d'intérêt peut être calculée si l'on connaît l'activité spécifique de chacun des marqueurs.

Comme on l'a indiqué, selon l'invention, la mesure du rapport des quantités d'ADN standards et à quantifier ne nécessite pas obligatoirement leur séparation physique, en particulier par électrophorèse.

En particulier, dans ces derniers modes de réalisation :

A l'étape 2), les amorces d'amplification peuvent être modifiées chimiquement de manière à pouvoir être liées à une phase solide et après l'étape 3), on lie les produits d'amplification à la phase solide par l'intermédiaire desdites amorces modifiées, puis la phase solide est lavée de manière à éliminer les amorces marquées en excès de l'étape 3) n'ayant pas réagi.

Bien entendu, dans ce dernier mode de réalisation, si les fragments d'ADN marqué d'intérêt et standard ne différent qu'en un seul site, les deux oligonucléotides amorces de l'étape 3) incluent ce site.

Dans un mode de réalisation, les amorces d'amplification à l'étape 2) sont biotinylées et peuvent réagir et se lier avec la streptavidine, laquelle est couplée à la phase solide.

Selon une variante de réalisation, la phase solide peut être constituée par des billes magnétiques. Un champ magnétique permet une sédimentation rapide des produits amplifiés pendant l'étape de lavage de la phase solide.

Pour assurer une bonne détection du signal, il est préférable que si $X_o$ représente la quantité initiale du fragment d'ADN standard et $Y_o$ celle des fragments d'ADN d'intérêt, ils répondent à la formule

$$10.X_o \rangle Y_o \rangle \frac{1}{10} . X_o.$$

C'est pourquoi, de préférence, on réitère le procédé selon l'invention plusieurs fois dans différents tubes, dans les mêmes conditions, excepté les quantités de fragments d'ADN standards initiales variables selon les tubes.

Le procédé selon l'invention est applicable à tout procédé impliquant l'utilisation des méthodes d'amplification d'ADN, notamment les procédés de diagnostic.

La présente invention a enfin pour objet un kit de détermination de la quantité d'un fragment d'ADN d'intérêt par un procédé selon l'invention, caractérisé en ce qu'il comporte :

- un fragment d'ADN standard ne différant en taille et/ou en séquence de pas plus de 10% environ, de préférence de pas plus de 5 nucléotides par brin du fragment d'ADN d'intérêt ;
- des réactifs pour la mise en oeuvre de ladite méthode d'amplification enzymatique in vitro et notamment des oligonucléotides amorces ;
- un ou plusieurs oligonucléotide(s) sondes ou amorces marqué(s), qui s'hybride(nt) spécifiquement avec les fragments d'ADN standard et d'intérêt, le cas échéant, à un site approprié pour qu'une élongation par l'ADN polymérase génère des fragments d'ADN marqués de tailles et/ou de séquences différentes ou avec des marqueurs différents selon qu'ils proviennent des fragments d'ADN d'intérêt et standard respectivement.

D'autres avantages et caractéristiques de la présente invention apparaîtront à la lumière de la description qui va suivre.

Les Figures 1 à 4 illustrent l'exemple 1.

La Figure 1 représente les séquences de la partie 3' du gène H-2 $K^d$ (PH-2) (gène de classe I du complexe majeur d'histocompatibilité (CMH), de l'ADN standard (PH-2$_{stand}$) et des amorces d'amplification (H2-I et H2-II) et de la troisième amorce marquée (H.2-III).

La Figure 2a montre la résolution de la séparation électrophorétique de deux fragments ampliés différents, dans des concentrations initiales variables.

La Figure 2b représente la courbe de quantification de H-2 $K^d$ par l'ADN standard.

La Figure 3 représente la courbe de quantification des ADNC transcrits de gènes de classe I du CMH dans différents organes.

La Figure 4 représente l'augmentation des transcrits de gène de classe I du CMH dans un foie en phase aiguë.

La Figure 5 représente les séquences des ADNc du gène TcRbêta de BW5147 et du clone K17.

La Figure 6 représente les résultats de l'exemple 2. Nombre de copies du plasmide ajoutées : a) pas de plasmide, b) 300 copies, c) 3000 copies, d) 30000 copies, e) 300000 copies

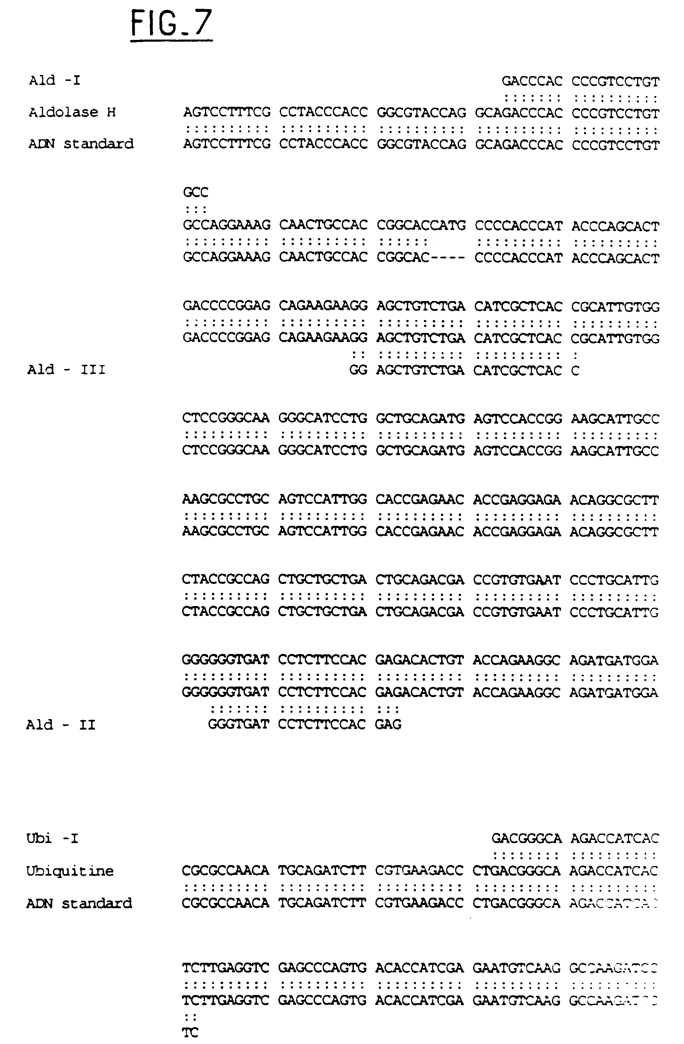

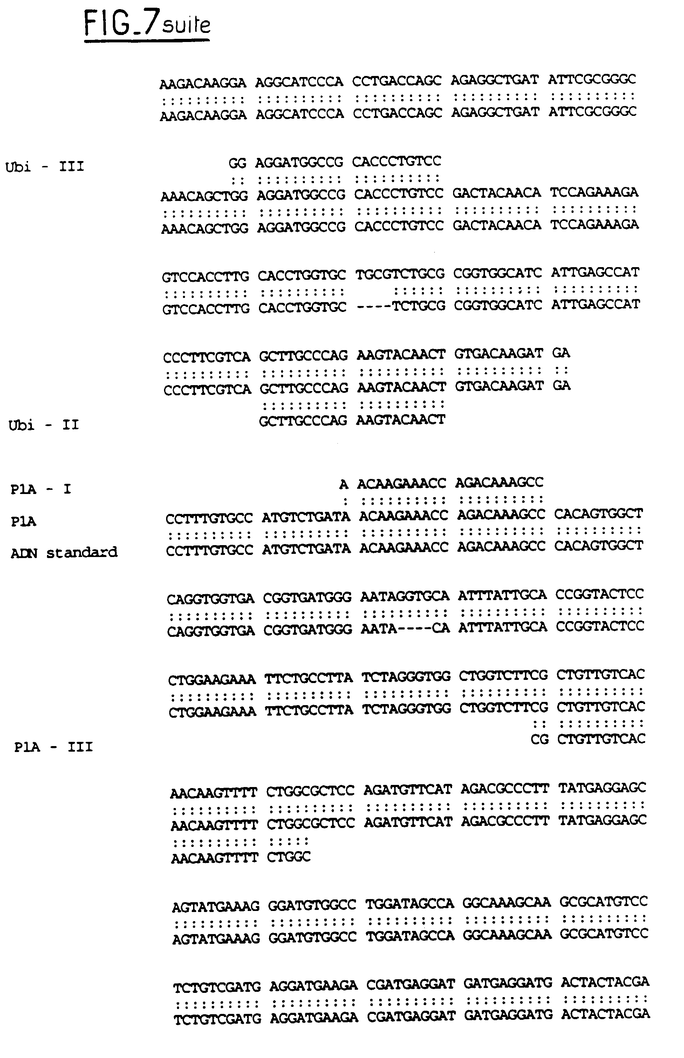

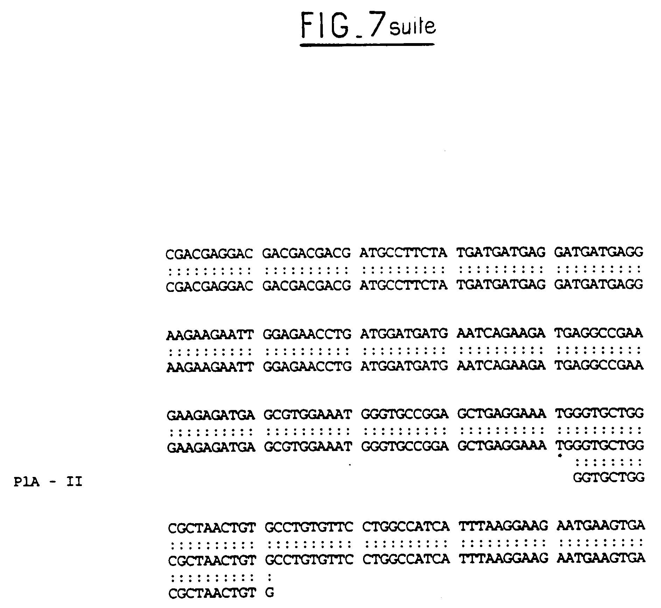

La Figure 7 représente les séquences des amorces d'ADN d'intérêt et ADN standards de l'Exemple 4.

Les figures 8 à 11 illustrent l'exemple 5 qui est un exemple comparatif et récapitulatif.

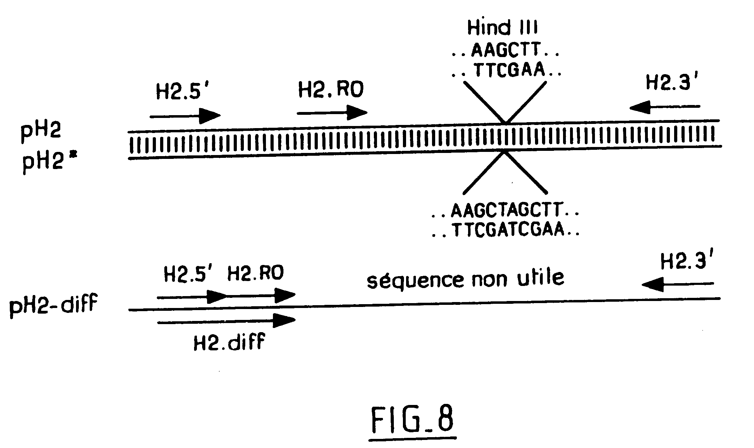

La figure 8 représente la construction de deux plasmides étalons pour la quantification d'ARNm de H-2K.

pH2* a été dérivé de pH2 par insertion de 4 paires de bases au site HindIII, pH2-diff a été construit par clonage d'un produit de PCR non spécifique engendré avec des amorces H2-diff et H2-3'. En raison de la séquence de H2-diff, pH2-diff peut toujours être amplifié par H2.5' et H2.3' et détecté à l'aide d'H2.RO dans une expérience d'amplification complémentaire.

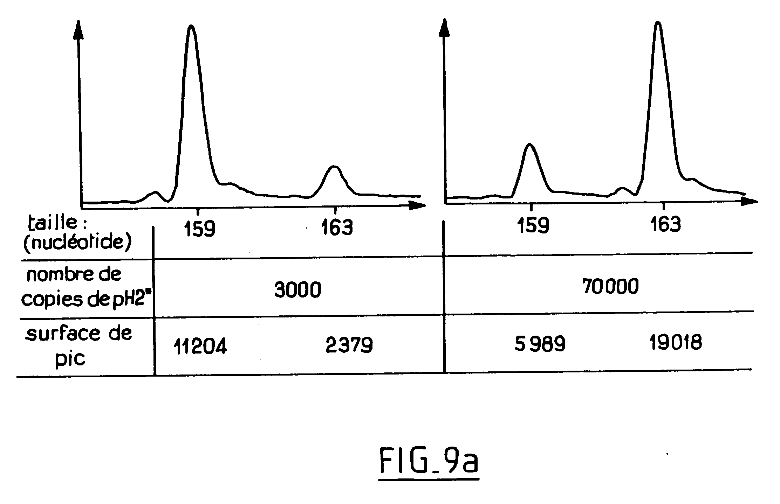

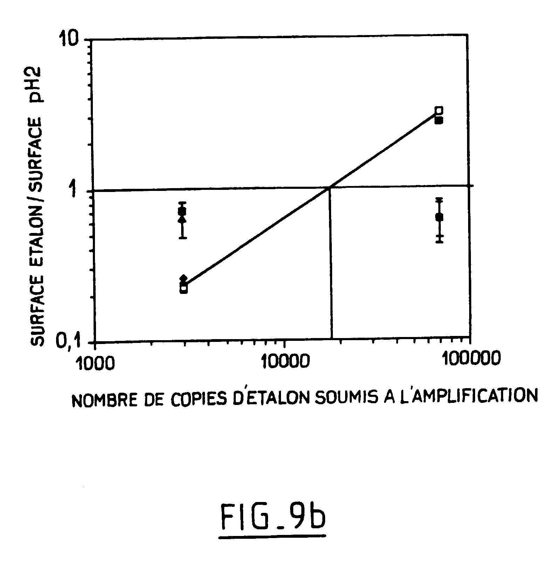

Les figures 9a et 9b représentent la quantification de pH2 à l'aide de pH2* et pH2-diff comme ADN étalon.

Environ $10^4$ copies de pH2 ont été co-amplifiées à l'aide de H2.5' et H2.3' en même temps que $3.10^3$ ou $7.10^4$ copies soit de pH2∗ soit de pH2-diff. Une réaction d'amplification complémentaire a été effectuée sur la matière amplifiée à l'aide de l'oligonucléotide fluorescent HI.RO. 25 ou 30 cycles ont été réalisés, le plateau étant atteint après 25 cycles. A - Après électrophorèse, la surface du pic pour chacune des deux espèces de l'amplification complémentaire a etc mesurée dans chaque échantillon. B - Le rapport a été calculé et porté en fonction du nombre de copies d'ADN étalon mélangée avec pH2. Toutes les expériences ont été réalisées en double. Pour chaque point, un écart type est indiqué. Le nombre de copies de pH2 a été déterminé comme étant l'abscisse du point de la courbe dont l'ordonnée est égale à 1. ☐ pH2-étalon-25 cycles, ◆ pH2-étalon-30 cycles, ■ pH2 - diff-25 cycles ▲ pH2-diff - 30 cycles.

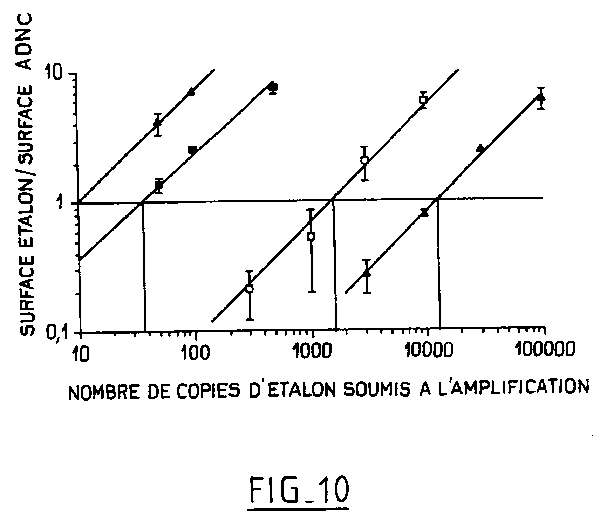

La figure 10 représente la quantification d'un ARNm spécifique de la chaine β du récepteur des cellules T de souris au cours d'une immunisation.

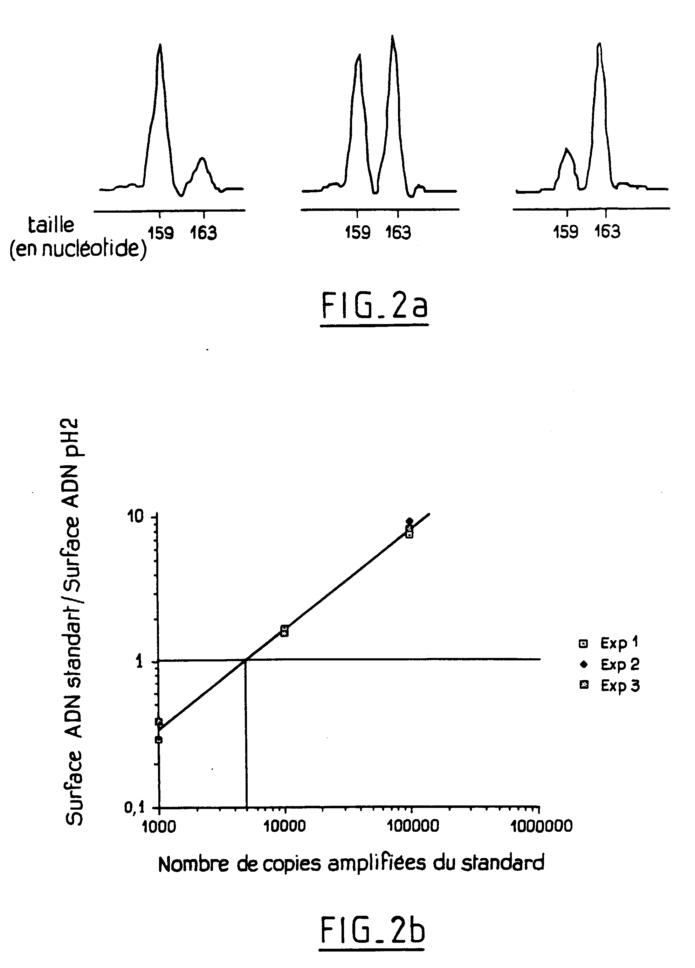

Les ARN totaux des ganglions lymphatiques de souris B10.A immunisée deux fois (D14) ou hyperimmunisée (D49) soit avec l'adjuvant complet de Freund (CFA) seul soit avec la cytochrome C de pigeon (PCC) mélangée avec du CFA ont été préparés et transcrits par la transcriptase inverse en ADNc (30). Différentes dilutions de l'ADN étalon pVβ3* ont été mélangées avec une quantité constante des différents ADNc, correspondant à la transcription inverse de 200 ng d'ARN total et amplifiés jusqu'à saturation (40 cycles) avec les oligonucléotides Vβ3.5' et Jβ1.2.3. Une réaction d'amplification complémentaire a été effectuée sur chaque matière amplifiée à l'aide de l'oligonucléotide fluorescent Vβ3.RO. Comme pour la figure 2, l'électrophorère a été effectuée, les surfaces de pics mesurées et les rapports ont été portés en fonction du nombre d'ADN étalon pVβ3* mélangé avec les différents ADNc. Le nombre de copies a ensuite été déterminé comme à la figure 2. ▲ D14 après immunisation avec CFA, ■ D49 après immunisation avec CFA □ D14 après immunisation avec PCC, ▲ D49 après immunisation avec PCC.

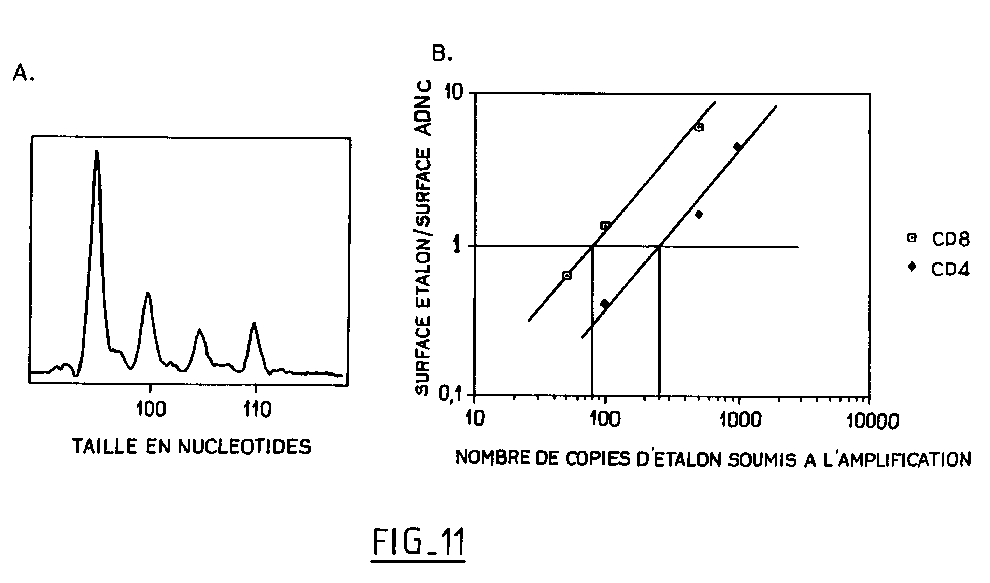

La figure 11 représente l'analyse simultanée de plusieurs ARNm dans une seule préparation d'ADNc.

A - Exemple des pics de fluorescence obtenus en déposant simultanément des produits de l'amplification complémentaire à partir de la co-amplification par PCR de 20 ng d'ARNm d'un thymus de souris avec soit 100 copies de pCD4*, soit 500 copies de pCD8*. Les produits ont une longueur de 96 pour pCD4*, 100 pour l'ARNm de CD4, 106 pour pCD8* et 110 pour l'ARNm de CD8 et ils peuvent être facilement identifiés.

B - Quantification de l'ARNm de CD4 et de CD8 dans 20ng d'ARNm total d'un thymus de souris. Les quantités sont calculées comme précédemment.

## **EXEMPLE 1** :

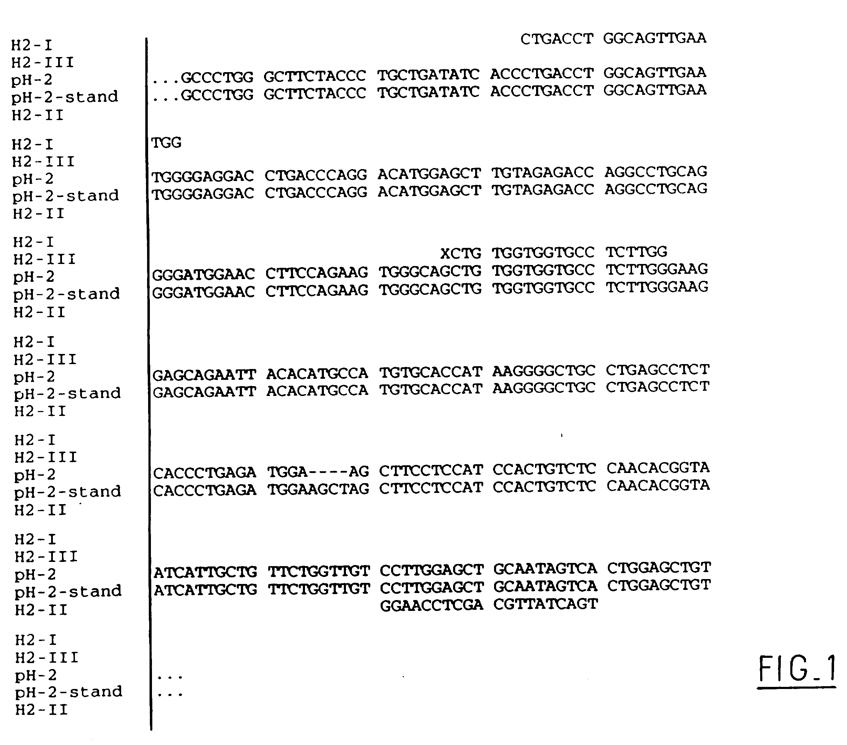

Dans cet exemple, on mesure la concentration des ARN messagers résultant de la transcription des gènes de classe I du Complexe Majeur d'Histocompatibilité de la souris, dans différents organes et dans différentes conditions expérimentales. Pour ce faire, on construit un ADN standard à partir de la séquence du gène H- ZK$^d$ (Figure 1). Cet ADN standard est ensuite utilisé dans les expériences d'amplification subséquentes comme échantillon standard.

MATERIEL ET METHODES :

### **1. Souris, souche bactérienne et vecteurs.**

Les souris BALB/c proviennent de l'animalerie de l'institut Pasteur.

Les ADN d'intérêt sont insérés dans le plasmide Bluescript pB5 SK*. Les plasmides recombinants obtenus sont clonés par électroporation de la souche E. Coli DH5alpha (Woodcock et al, 1989) selon le protocole décrit par Dower et al. (1988). Le plasmide pH-2$^d$-33 a été construit par Lalanne et al. (1983).

### **2. Oligonucléotides amorces synthétiques.**

Les oligonucléotides synthétiques ont été synthétisés en utilisant un synthétiseur Applied Biosystem 381A et les réactifs correspondants. L'amorce fluorescente a été marquée en utilisant les procédures et les réactifs d'Applied Biosystem (synthèse avec aminolink à l'extrémité 5' de l'oligonucléotide, suivie de l'étape de marquage proprement dite). Seul le fluophore Fam a été utilisé. La liste des oligonucléotides est donnée ci-dessous.

```
Oligonucléotide amorce H2-I:     5'-CTGACCTGGCAGTTGAATGG-3'

Oligonucléotide amorce H2-II:    5'-TGACTATTGCAGCTCCAAGG-3'

Oligonucléotide amorce marqué  H2-III:
                                 5'-XCTGTGGTGGTGCCTCTTGG-3'
```

où X désigne le marqueur fluorescent.

### 3. Extraction d'ARN.

Les ARN ont été préparés en utilisant le protocole décrit par Chrigwin et al. (1979) : le tissu d'intérêt est broyé dans 7 ml d'une solution d'isothiocyanate de guanidine 4M contenant 0,5% de Na N-laurylsarcosine, 2 mM EDTA, 0,13% d'anti-foam A, et déposé sur un gradient discontinu de Chlorure de Césium composé de 3 ml d'une solution 5,7 M de CsCl, 25 mM Na-Acétate pH 5,2, 10 mM EDTA, recouverts par 0,7 ml d'une solution 2,4 M CsCl, 25 mM Na-Acétate pH 5,2, 10 mM EDTA. L'ensemble est ensuite ultracentrifugé 24 heures à 30 000 tr/mn à 20°C dans un rotor SW41. Aprés décantation, le culot est resuspendu dans 400 μl d'une solution 10 mM Tris-HCl, pH 7,5, 1 mM EDTA, 5% Na N-laurylsarcosine, 0,1 M NaCl, 5°o phénol. L'ARN est ensuite extrait par addition d'un volume de phénol et d'un volume de chloroforme. La phase aqueuse est enfin précipitée à l'éthanol et le culot dissout dans de l'eau.

### 4. Transcription inverse.

La synthèse d'ADN complémentaire est réalisée selon le protocole et en utilisant les réactifs fournis dans le kit de transcription inverse de Boehringer. En particulier, l'oligonucléotide utilisé comme amorce de la transcription est un 15-mer poly-dT. 10 μg d'ARN total sont utilisés dans chaque expérience de transcription inverse.

### 5. Amplification par la méthode PCR.

Les amplifications sont réalisées dans 50 μl d'une solution contenant 20 u/ml de l'enzyme polymérase Taq (Proméga[R]), 200 μM de chacun des nucléotides triphosphates dATP, dGTP, dCTP et dTTP, 0,5 μM de chacun des oligonucléotides H2-I et H2-II, dans le tampon Proméga, le tout recouvert de 50 μl d'huile minérale. Les automates perkin-Elmer[R] et Prem de Lepscientific[R] ont été utilisés. Les conditions d'amplification sont les suivantes : l'échantillon est tout d'abord chauffé 1 mn à 94°C puis soumis à un certain nombre cycles du programme suivant : 1 mn à 94°C, 1 mn à 60°C et 4 mn à 72°C. Enfin, l'échantillon est amené à 72°C pendant 10 mn. La concentration d'ion Mg$^{2+}$ utilisée est 1,5 mM

### 6. Elongation supplémentaire.

Un aliquot de 2 μl du milieu de réaction PCR précédent est utilisé dans une expérience de "run-off". Cette réaction est réalisée dans un volume de 10 μl d'une solution contenant 0,1 μM de l'amorce marquée fluorescente H2-III, 20 u/ml de l'enzyme polymérase Taq (Proméga[R]), 200 μM de chacun des nucléotides triphosphates dATP, dGTP, dCTP et dTTP, dans le tampon Proméga[R], le tout recouvert de 20 μl d'huile minérale. La concentration d'ion Mg$^{2+}$ utilisée est 1,5 mM. Le programme de l'élongation est le suivant : après une étape de dénaturation de 2 mn à 94°C, l'échantillon est porté à 60°C pendant 1 minute, puis à 72°C pendant 15 mn. Cette réaction est réalisée en utilisant les mêmes automates que pour les réactions PCR.

### 7. Electrophorèse.

Les produits fluorescents de l'étape d'élongation sont séparés selon leur taille dans un gel séquenceur d'ADN en utilisant le dispositif Applied Biosystems 373A[R]. Le gel a une épaisseur de 400 μm et a une concentration de 4% d'acrylamide et 8M urée. Les échantillons sont mélangés à 1 volume d'une solution 99% formamide, 20 mM EDTA, et dénaturés pendant 10 mn à 80°C. 2 μl de cette solution sont alors chargés sur le gel. L'électrophorèse est réalisée à 30W pendant 3 heures.

### 8. Analyse des données d'électrophorèse.

Un logiciel approprié est employé pour analyser les données enregistrées par le dispositif Applied Biosystems 373A. Les caractéristiques essentielles de ce logiciel sont de permettre de mesurer les surfaces des pics correspondants avec une précision relative inférieure à 5%, et de déterminer les tailles de molécule d'ADN simple brin à ± 0,2 nucléotide près par rapport à des standards de taille ad hoc.

RESULTATS

### 1. Construction de l'ADN standard.

On a construit deux plasmides à partir de pH-2$^d$-33. Le fragment KpnI-DraI du plasmide pH-2$^d$-33 a été introduit dans le vecteur Bluescript pBS SK$^+$ aux sites KpnI et EcoRV. pH2, le plasmide recombinant ainsi obtenu contient la partie 3' de l'ADN complémentaire codant pour l'antigène d'histocompatibilité H-2K$^d$. A partir de ce plasmide, on construit le plasmide pH2-stand en digérant pH2 au site HindIII et en rendant franches les extrémités du plasmide linéarisé par la polymérase du phage T4. Une fois religué et cloné, ce plasmide diffère donc de pH2 par l'addition de 4 paires de bases au site HindIII (voir Figure 1).

Enfin, on a synthétisé trois oligonucléotides s'hybridant dans des régions très conservées des gènes H-2 de Classe I.

## 2. Détermination du seuil de saturation.

Dans une première série d'expérience, on a déterminé, pour une certaine quantité d'ADN initiale, le nombre de cycles d'amplification au delà duquel la quantité d'ADN cesse de croître de façon exponentielle en fonction du nombre de cycles d'amplification.

On a donc amplifié, dans des expériences parallèles, des quantités variables du plasmide pH2-stand. Les quantités d'ADN varient de $10^2$ à $10^8$ copies de plasmide, et le nombre de cycles d'amplification de 20 à 45 cycles.

On a montré que le seuil au delà duquel l'amplification cesse d'être exponentielle, est variable, même si la concentration initiale d'ADN est la même. Cependant, dans les conditions expérimentales décrites, lorsque plus de 1000 copies du plasmide pH2-stand sont amplifiées, ce seuil est atteint dans tous les cas avant 30 cycles d'amplification. Dans toutes les expériences rapportées par la suite, toutes les amplifications sont réalisées "à saturation", puisqu'on amplifie pendant 40 cycles plus de 1000 copies de l'ADN standard.

## 3. Quantification d'un plasmide H-2K$^d$ par l'ADN standard.

On a ensuite montré que le protocole décrit permet effectivement de déterminer la concentration du plasmide pH2 en connaissant la concentration de l'ADN standard pH2-stand.

Pour ce faire, on a mélangé environ 7000 copies du plasmide pH2 (obtenues après dilution d'une solution dont la concentration est déterminée par mesure de la densité optique à 260 nm) à 1000, 10000 et 100000 copies du plasmide pH2-stand. Ces différents mélanges ont été amplifiés et les résultats sont reportés sur la Figure 2; la Figure 2a) montre que la résolution de la séparation électrophorétique est suffisante pour séparer les produits de l'élongation issus des deux espèces amplifiées. Pour un même échantillon amplifié dans des expériences différentes, les surfaces de chacun des pics varient d'une expérience à l'autre alors que les rapports entre ces surfaces sont remarquablement reproductibles. La Figure 2b) montre qu'il est alors aisé de déterminer la concentration initiale du plasmide pH2. Des expériences similaires ont été réalisées en variant le nombre de copies du plasmide pH2 de 100 à 1000000 de copies. Des résultats identiques ont été obtenus, sauf lorsque le nombre de copies est trop faible : dans ce cas, la quantification est moins fiable.

## 4. Quantification de la concentration des transcrits des gènes de classe I du CMH dans différents organes.

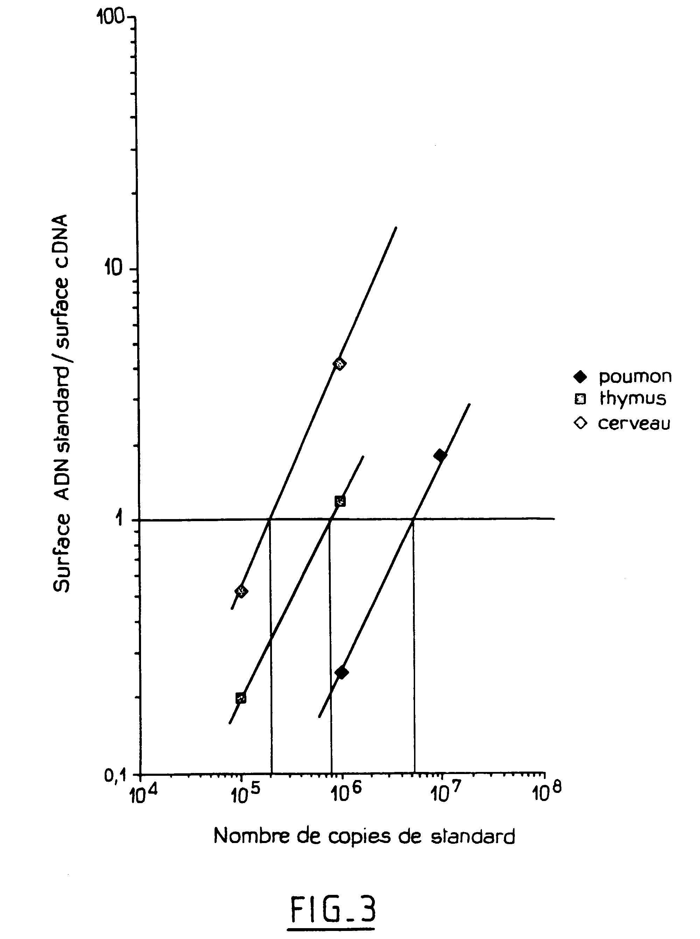

On a alors appliqué cette méthode à la détermination du nombre de copies d'ADN complémentaire présents après la transcription inverse d'une quantité connue d'ARN total d'un certain nombre de tissus. Pour cela, on a préparé l'ARN de divers tissus de souris BALB/c (haplotype H-2$^d$). 10 µg d'ARN total ont été utilisés pour la synthèse d'ADN complémentaire. Puis, 1/50e de ces ADN complémentaires a été mélangé à des quantités variables du plasmide pH2-stand. Les résultats de la quantification à partir de l'ARN de quelques organes sont montrés à la Figure 3. Dans cette expérience, l'ADN complémentaire synthétisé est un ADN monocaténaire, alors que l'ADN standard est un ADN bicaténaire. Pour tenir compte de cette différence, les concentrations obtenues ont été multipliées par deux.

Les nombres de copies d'ADN complémentaire présents après la transcription inverse de 100 ng d'ARN total sont donc :

dans le poumon : 50.10$^5$ copies,
dans le thymus : 8.10$^5$ copies,
dans le cerveau : 2.10$^5$ copies.

## 5. Mesure de l'augmentation de la concentration des transcrits des gènes de Classe I du CMH dans le foie après induction d'une phase aiguë.

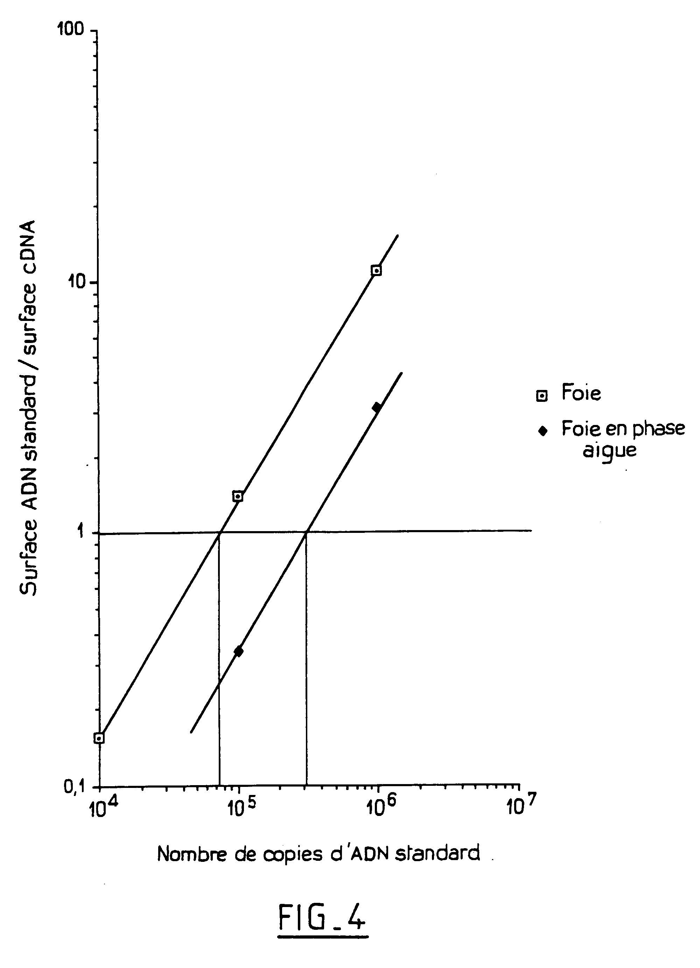

On a enfin comparé la concentration d'ADN complémentaires des transcrits des gènes H-2 de classe I dans un foie de souris BALB/c en phase aiguë 24 heures après l'injection de LPS, à la concentration de ces ADN complémentaire dans le foie d'une souris témoin. Les résultats de la quantification de ces ADN complémentaires sont montrés dans la Figure 4. Une variation d'un facteur 3 est aisément détectée.

## EXEMPLE 2 :

De l'ARN a été extrait d'une tumeur de cellules T (BW5147 Barth et al., 1985) dont le récepteur de cellule T a été caractérisé par séquençage des ARNm codant les chaînes alpha et bêta. Un ADNc monobrin a été synthétisé et utilisé comme fragment d'ADN d'intérêt dans des amplifications subséquentes. On souhaitait quantifier le nombre de transcrits codant la chaîne bèta de ce récepteur par cellule.

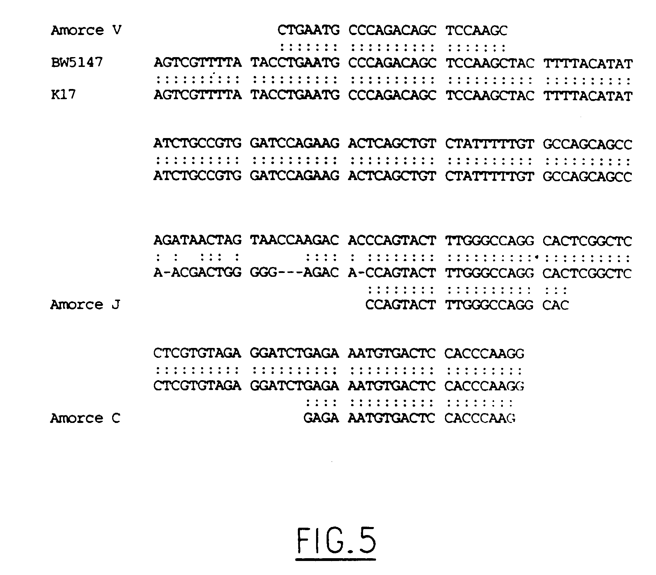

Ici, au lieu d'utiliser un ADNc à séquence mutée comme échantillon interne standard, on a utilisé un ADNc déjà cloné dans le plasmide K17 qui donne des produits d'élongation plus petits de 5 nucléotides que l'ADNc à quantifier (séquence BW 5147) et qui en diffère en taille de 5 nucléotides et en séquence de 11 nucléotides. La séquence du fragment d'ADN à quantifier (BW 5147) faisait plus de 170 nucléotides. Les séquences comparées de BW 5147 et K17 sont données dans la Figure 5.

Des quantités variées de standard (K17) ont été ajoutées, mélangées et co-amplifiées avec l'ADNc à quantifier jusqu'à saturation obtenue après 40 cycles à l'aide des amorces V et C.

Un aliquot de ce milieu d'amplification a été utilisé comme matrice dans la réaction d'élongation avec la troisième amorce J commune aux fragments d'ADN standards et d'intérêt.

On a séparé physiquement, par électrophorèse sur gel, les fragments d'ADN marqués provenant des fragments d'ADN standards et d'intérêt (ADNc) et on a mesuré le rapport des quantités des fragments d'ADN standard et d'intérêt en mesurant l'intensité du signal (surface des pics de fluorescence) correspondant aux deux types de fragments.

## MATERIEL ET METHODES

### 1. Souche bactérienne et vecteurs

Les ADN d'intérêt sont constitués par des ADNc correspondant à des transcrits de gènes codant pour la chaîne bèta des récepteurs de cellules T murins.

Le plasmide K17 contient le fragment d'ADN standard et a été construit à partir du vecteur M13m p18 (Biolabo) qui a été rendu en ADN monobrin dans la capside du bactériophage m13.

### 2. Les oligonucléotides synthétiques

Les oligonucléotides amorces ont été synthétisés en utilisant un synthétiseur Applied Biosystem 381A et les réactifs correspondants. Les amorces fluorescentes ont été marquées en utilisant les procédures et réactifs d'Applied Biosystem. Le fluorophore Fam a été la plupart du temps utilisé mais trois autres ont été également utilisés avec des résultats similaires. La liste des oligonucléotides est donnée ci-dessous.

V : CTGAATGCCCAGACAGCTCCAAGC

C : CTTGGGTGGAGTCACATTTCTC

J : XGTGCCTGGCCCAAAGTACTGG

où X est le marqueur fluorescent.

### 3. Méthode PCR

Les amplifications ont été réalisées sur les automates Perkin Elmer Cetus et Prem, selon la technique décrite (Saiki et al., 1988).

Pour amplifier l'ADN complémentaire, le protocole utilisé fut le suivant : 5 µl de la solution préparée précédemment sont amplifiés dans 50 ou 100 µl d'une solution 50 mM KCl, 10 mM Tris-HCl, pH 8.2, 0,01°o gélatine, 0,5 µM en chacun des deux oligonucléotides V et C, 2 u par 100 µl de Taq Polymérase (Beckman), 200 µM en chacun des nucléotides dATP , dGTP, dCTP et dTTP. Le tout est recouvert de 50 µl d'huile minérale, chauffé 1 mn à 94°C, amplifié par 40 cycles (1 mn à 94°C, 1 mn à 65°C. 1 mn à 72°C puis incubé 10 mn à 72°C.

### 4. Elongation supplémentaire

Un aliquot de 2 μl de milieu de réaction PCR précédent a été utilisé dans une expérience de "run off" ; l'amorce marquée fluorescente (Jbèta 2.5) a été ajoutée à la concentration finale de 0,1 μM dans le même mélange que celui utilisé pour l'amplification.

L'amorce J est donc dans un excès par rapport aux autres amorces V et C apportées par le μl du mélange amplifié. La réaction de "run off" consiste en 2 mn à 94°C, 1 mn à 60°C suivi de 15 mn à 72°C.

### 5. Séparation des produits amplifiés selon leur taille

Les produits amplifiés marqués avec le marqueur fluorescent ont été séparés selon leur taille dans un gel séquenceur en utilisant le dispositif Applied Biosystems 373A avec séquenceur d'ADN automatisé. On a utilisé un logiciel approprié permettant de numériser les pics de fluorescence enregistrés.

### 6. Electrophorèses

L'analyse des produits d'amplification et des séquences est réalisée par une électrophorèse sur gel de 400 μm d'épaisseur, de 6°o polyacrylamide (réticulation : 1 g de bisacrylamide pour 19 g d'acrylamide) dans le tampon TBE (Maniatis et al., 1982) en conditions de dénaturation (95% de formamide, 20 mM EDTA, et incubés 10 mn à 80°C. La migration est réalisée dans le tampon TBE sous un champ électrique de 4 000 Vm$^{-1}$, 5 à 6 h pour l'analyse de produits de l'étape de "Run off".

### EXEMPLE 3 :

Une prise de sang de 1 millilitre a été réalisée sur une souris BALB/c. Dans cette prise de sang sont présents de nombreux lymphocytes T qui expriment à leur surface des récepteurs (TCR) impliqués dans la reconnaissance des antigènes par ces cellules. Les deux gènes codant pour les chaînes alpha et bèta de ce récepteur sont constitués à partir de segments de gènes (appelés V, D, J et C), réarrangés lors d'une des étapes de maturation de la cellule. Les réarrangements s'opèrent de telle sorte que les jonctions entre les segments V et J ont des séquences dont la longueur peut varier de quelques nucléotides. Cette longueur (variable de cellule à cellule) est appelée "longueur de la région CDR3". On souhaitait quantifier le nombre de transcrits codant pour la chaîne bèta de ce récepteur par μg d'ARN extrait, lorsque le gène codant pour cette chaîne bèta utilise certains segments V et J, pris parmi les segments disponibles dans le génome de la souris. Plus précisément, on détermine ici le nombre de transcrits codant pour des chaînes bèta utilisant les segments géniques Vbèta 6 et Jbètal,5, et ayant une certaine longueur de la région CDR3. Un ADNc double brin a été synthétisé, et utilisé dans les amplifications subséquentes.

On utilise comme échantillon interne standard un ADNc codant pour l'une de ces chaînes, cloné dans un plasmide. Cette chaîne a une longueur de la région CDR3 égale à 7 acides aminés.

Des quantités variables de ce plasmide ont été ajoutées, mélangées et co-amplifiées avec l'ADNc à quantifier jusqu'à saturation obtenue après 40 cycles à l'aide des amorces V et C.

Un aliquot de ce milieu d'amplification a été utilisé comme matrice dans la réaction d'élongation avec la troisième amorce J commune aux fragments d'ADN et d'intérêt.

On a séparé physiquement par électrophorèse sur gel, selon le même protocole qu'à l'exemple 1.

### MATERIEL ET METHODES :

1. L'ADNc constituant le standard est cloné dans le vecteur Bluescript pBS SK$^+$, en utilisant comme hôte une souche E. Coli XL1 Blue.

2. La liste des oligonucléotides utilisés est donnée ci-dessous.

V: CTCTCACTGTGACATCTGCCC

J: XGAGTCCCCTCTCCAAAAAGCG

C: CTTGGGTGGAGTCACATTTCTC

où X est le marqueur fluorescent.

3. Le reste des méthodes est identique à celles mises en oeuvre dans l'Exemple 1.

RESULTATS

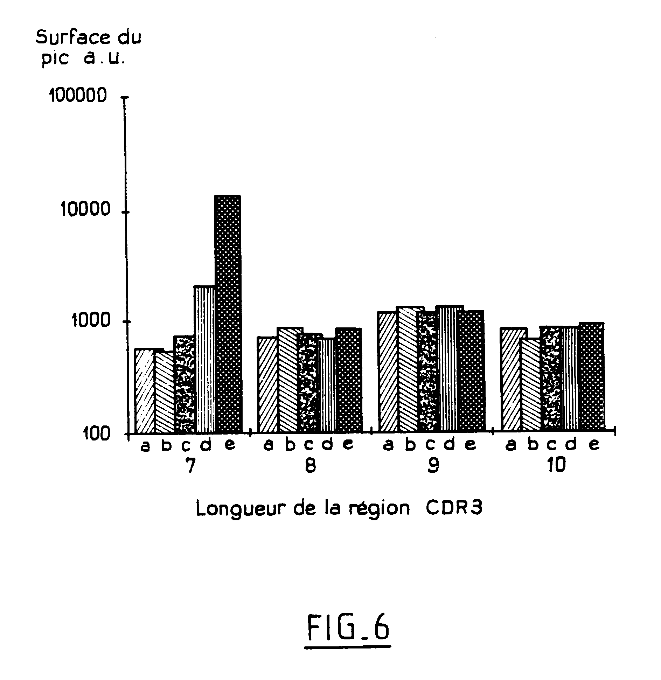

La Figure 6 montre que:

- dans la population de lymphocytes T prélevés lors de la prise de sang, il s'en trouve qui ont des récepteurs T dont la chaîne bèta utilise les segments Vbèta6 et Jbèta 1,5 et dont la longueur de la région CDR3 varie de 7 à 10 acides aminés.
- d'autre part, lorsque moins de 300 copies du plasmide contenant l'ADN standard sont ajoutées avant amplification, la quantité d'ADN amplifiés ayant une longueur du CDR3 égale à 7 acides aminés demeure inchangée, à la précision des mesures près, par rapport à la quantité d'ADN ayant une longueur de la région CDR3 différente de 7. Par contre, lorsque 3000 copies et plus de l'ADN standard sont ajoutées, une contribution due à l'ADN standard est observée dans le signal généré par les ADNc ayant une longueur du CDR3 égale à 7. Cette contribution relative au signal est calculée en soustrayant du signal total, le signal obtenu lorsqu'aucun ADN standard n'est ajouté. Le tableau 2 montre que cette contribution varie dans le même rapport que le nombre de copies ajoutées de l'ADN standard (comme 1 et 3 du tableau).

Lors de chacune de ces amplifications, l'ADNc obtenu à partir de la transcription inverse de 100 ng d'ARN extrait est utilisé. On estime donc que le nombre de transcrits présents dans ces 100 ng d'ARN total, codant pour des chaînes bèta utilisant les segments VbètaH6 et Jbèta1,5 et ayant de plus, une longueur du CDR3 égale à 7 est de 3000 x 560 / 140 soit: 12000 copies. On peut de même déduire la quantité de ces transcrits dont la longueur du CDR3 vaut 8, 9 ou 10 acides aminés.

TABLEAU 2

| Nombre de copies du plasmide | Intensité du pic de 7 aa | Signal généré par le plasmide |
|---|---|---|
| 0 | 560 | 0 |
| 300 | 530 | 0 |
| 3000 | 700 | 140 |
| 30000 | 1970 | 1410 |
| 300000 | 12940 | 12380 |

**EXEMPLE 4** :

Un autre avantage pratique du procédé selon l'invention est de permettre, pour différents échantillons pour lesquels les amorces et étalons standards sont espacés de façon appropriée, d'effectuer l'analyse sur un même gel. On a pu ainsi développer un test qui permet des mesures simultanées d'ARNm codant pour une variété d'oncogènes pour la caractérisation de tumeurs humaines ou murines.

Dans cet exemple, on mesure la concentration des ARN messagers résultant de la transcription de différents gènes murins dans des biopsies de tumeurs dérivant du mastocytome P815, chez des souris DBA/2 (Van den Eynde et al., 1991).

Pour cela, on synthétise les ADN complémentaires par transcription inverse d'une quantité déterminée (10 µg) d'ARN total préparé à partir de la biopsie. Dans cette population d'ADN complémentaires, on quantifie selon la méthode décrite dans la présente invention le nombre de copies des ADNc codant pour différents gènes d'intérêt.

Les transcrits d'intérêt sont : l'aldolase H (Mestek et al., 1987; Maire et al., 1987) et l'ubiquitine dont les gènes sont exprimés de façon ubiquitaire, les ARN messagers du gène PIA codant pour un des déterminants antigéniques de la tumeur P815 (Van den Eynde et al., 1991), et les ARN messagers des gènes de Classe I du Complexe Majeur d'Histocompatibilité déjà décrit dans l'Exemple 1. Pour chacun de ces 6 gènes (ou familles de gènes), on synthétise un ADN plasmidique comme décrit dans les Exemples 1 et 2. Ces 4 ADN standards sont utilisés dans les amplifications subséquentes pour déterminer le nombre de copies d'ADN complémentaire pour chacun de ces 4 (familles de) gènes, comme décrit dans l'exemple 1.

Les oligonucléotides sont synthétisés de telle façon que les produits d'extension fluorescents issus de ces 4 quantifications, une fois mélangés, puissent être analysés simultanément sur une même piste du gel d'électrophorèse.

Les séquences des oligonucléotides utilisés sont données ci-dessous, ainsi que les tailles en nucléotides des produits de l'amplification et de l'élongation supplémentaire. La nomenclature utilisée pour la désignation des amorces est la même que celle employée dans l'Exemple 1 : pour chaque gène, les amorces I et II sont utilisées dans l'amplification, tandis que l'amorce III est fluorescente et utilisée dans l'élongation supplémentaire.

Liste des oligonucléotides utilisés:

| Séquence 5' vers 3' | | Taille du produit d'amplification | Taille du produit d'élongation |
|---|---|---|---|
| **gène aldolase H:** | | 290 | 108 |
| **Ald - I:** | GACCCACCCCGTCCTGTGCC | | |
| **Ald - II:** | CTCGTGGAAGAGGATCACCC | | |
| **Ald -III:** | XGGTGAGCGATGTCAGACAGCTCC | | |
| **gène ubiquitine:** | | 247 | 122 |
| **Ubi - I:** | GACGGGCAAGACCATCACTC | | |
| **Ubi - II:** | AGTTGTACTTCTGGGCAAGC | | |
| **Ubi -III:** | XGGAGGATGGCCGCACCCTGTCC | | |
| **gène P1A:** | | 442 | 146 |
| **P1A - I:** | AACAAGAAACCAGACAAAGCC | | |
| **P1A - II:** | CACAGTTAGCGCCAGCACC | | |
| **P1A -III:** | XGCCAGAAAACTTGTTGTGACAACAGC | | |
| **gène H2 Classe I:** | | 253 | 159 |
| **H2 - I:** | CTGACCTGGCAGTTGAATGG | | |
| **H2 - II:** | TGACTATTGCAGCTCCAAGG | | |
| **H2 -III:** | XCTGTGGTGGTGCCTCTTGG | | |

Le procédé selon la présente invention s'est avéré, au cours d'un grand nombre d'expériences, étonnamment fiable, précis et reproductible. La possibilité d'effectuer la mesure à saturation simplifie les analyses simultanées d'échantillons multiples et augmente la sensibilité de détection à la fin de la réaction.

## EXEMPLE 5

Les fragments d'ADN à séquence connue (au moins en partie) peuvent être copiées in vitro à l'aide d'amorces oligonucléotidiques spécifiques et certaines ADN polymérases à la suite de procédés conduisant à une augmentation exponentielle du nombre de copies. La première technique, et celle la plus couramment utilisée, est la réaction de polymérase en chaîne (Polymerase Chain Reaction) (1, 2), mais d'autres techniques ont été décrites (3-6). Elles offrent un moyen très sensible de détection d'une séquence spécifique dans un mélange complexe d'acides nucléiques, permettant ainsi la détection de séquences d'ADN rares (par exemple une cellule infectée par des virus parmi plusieurs non-infectées) ou des transcrits d'ARN rares.

Toutefois, il s'est avéré difficile de rendre ces réactions d'amplification quantitatives, c'est-à-dire de déduire de manière précise, à partir des produits amplifiés, le nombre de séquences d'ADN présentes dans l'échantillon avant l'amplification. Cette quantification pourrait être une amélioration importante, par exemple dans la virémie à HIV ou dans l'évaluation du nombre de lymphocytes s'infiltrant dans une tumeur et, plus généralement, dans un grand nombre de tests de diagnostics et d'analyses médicales. Bien que diverses approches aient été développées au cours des dernières années (7-10), les meilleurs résultats ont apparemment été obtenus jusqu'à présent lorsqu'une molécule d'ADN de concentration connue est co-amplifiée avec les mêmes amorces dans le même tube et lorsque la réaction est arrêtée au cours de sa phase exponentielle bien avant qu'elle n'atteigne le plateau (11). De tels procédés sont actuellement imprécis et plutôt contraignants en pratique. La présente invention fournit des procédés quantitatifs faisant appel à des réactions conduites jusqu'au plateau. On décrit ici une méthode générale permettant la quantification après amplification menée jusqu'au plateau avec une précision allant jusqu'à ± 25%.

## MATERIEL ET METHODES

1) Les souris, les lignées cellulaires et les anticorps - les souris BALB/C ont été élevées localement. Ac38 est obtenu de la soucne CBA et résulte de la fusion avec X63Ag8 (H-2$^d$) (13), 42F8 est dérivé de cellules cliniques murines stimulées par le LPS fusionnées avec Sp$^{2/°}$ (H-2$^d$) et constitue un don de A Grandien (Institut Pasteur). X63Ag8.IL2 est dérivé de X63Ag8.653 (H-2$^d$) transfecté avec de l'ADNc d'IL2 (14) et constitue un don de F. Melchers (Institut d'Immunologie de Bâle). H97-76-7 est un anticorps monoclonal de souris renconnaissant la molécule K$^d$ de la classe I du CMH (15).

2) Cytométrie à flux - 5 x 10$^5$ cellules ont été culottées, lavées et remises en suspension dans 100 µl de PBS additionnés de 5% de sérum de veau foetal et incubées pendant 1 heure dans de la glace avec de l'H97-76-7 conjugué à PE. Les cellules ont été lavées et remises en suspension dans 100 µl du même tampon. La fluorescence rouge a été mesurée avec un cytomètre à flux FACSscan (Beckton Dickinson) avec le logiciel LYSIS II.

3) Les plasmides - pH-2$^d$-33 a été isolé par Lalanne et al. (16). pH2 résulte du clonage du fragment <u>Kpn</u>I-<u>Dra</u>I de pH-2$^d$-33 dans les sites <u>Kpn</u>I-<u>EcoR</u>V du plasmide Bluescript pBS SK$^+$. pH2* a été dérivé de pH-2 par digestion par <u>Hind</u>III suivie du remplissage par la T4 DNA polymérase des extrémités 3' sortantes qui sont produites et la ligation ultérieure du plasmide.

pH2-diff résulte du clonage d'un produit de PCR non spécifique, à l'aide des amorces H2.3' et H2.diff, dans le site <u>Sma</u>I de pBS SK$^+$ (figure 8).

pHIV était un don du Dr Wain-Hobson et contient la séquence mutant V4A.15 du gène TAT de HIV-I (17). pHIV* a été dérivé de pHIV par digestion par <u>Sac</u>I suivie de la coupure par la T4 DNA polymérase des extrémités 3' sortantes qui sont produites et la ligation ultérieure du plasmide.

pVβ3* contient la séquence de la région variable de la chaîne β du récepteur des cellules T du clone 5CC7 dans laquelle la séquence GATA d'une longueur de 4 paires de base a été insérée après le codon codant pour Ser 81 (18).

pCD4* et pCD8* résultent du clonage, dans le vecteur M13mp18 des produits de PCR engendrés respectivement par les amorces CD4.5' et CD4.3'ou CD8.5' et CD8.3' après clivage approprié par <u>EcoR</u>I et <u>Hind</u>III (pour pCD4) ou <u>EcoR</u>I et <u>Bam</u>HI (pour pCD8). Les recombinants ont alors été clivés respectivement par <u>Pst</u>I et <u>Stv</u>I, soumis à la nucléase d'haricots-mungo ou à la digestion par la T4 DNA polymérase pour engendrer des bouts francs et puis religués sur eux-mêmes, engendrant ainsi des vecteurs contenant les mêmes insertions avec une délétion de quatre bases, et désignés respectivement par pCD4* et pCD8*.

Ces vecteurs ont été vérifiés par des séquences simple brin (19).

pHPRT* a été obtenu par clonage du produit de PCR phosphorylé de HPRTdel et HPRT.3' dans M13mp18 précédemment clivé par <u>Sma</u>I et déphosphorylé.

4) Les oligonucléotides - les oligonucléotides suivants ont été utilisés:

| | |
|---|---|
| H2.5': | 5'-CTGACCTGGCAGTTGAATGG-3' |
| H2.3': | 5'-TGACTATTGCAGCTCCAAGG-3' |
| H2.RO: | 5'-XCTGTGGTGGTGCCTCTTGG-3' |
| H2.diff: | 5'-CTGACCTGGCAGTTGAATGCTGTGGTGGTGCCTCTTGG-3' |
| HIV.5': | 5'-CATTATTCAACAGAGGAGAGC-3' |
| HIV.3': | 5'-CTACTACTAATGCTACTATTGC-3' |
| HIV.RO: | 5'-XAAAGGCTTAGGCATCTCCTAT-3' |
| Vβ3.5': | 5'-GCAAAGATGAGGTGTATCCCTG-3' |
| Jβ.1.2.3: | 5'-GGTGTAGTCGGAGTTTGCAT-3' |
| Vβ3.RO: | 5'-XACCTTGCAGCCTAGAAATTCAGTCC-3' |

```
HPRT.5'  :  5'-GTAATGATCAGTCAACGGGGGAC-3'
HPRT.3'  :  5'-CCAGCAAGCTTGCAACCTTAACCA-3'
HPRTdel  :  5'-GTAATGATCAGTCAACGGGGGACATGTTATTGGTGGAGATGATCTCTCAA-3'
HPRT.RO  :  5'-XTTCTTTCCAGTTAAAGTTG-3'
CD4.5'   :  5'-CTGAATTCGGCGCTTGCTGCTGC-3'
CD4.3'   :  5'-CACAAGCTTAAGTCTGAGAGTCTTCC-3'
CD4.RO   :  5'-XTGCTGATTCCCCTTCCTTCC-3'
CD8.5'   :  5'-TAGAATCCTAGCTTGACCTAAGC-3'
CD8.3'   :  5'-ATGGATCCATATAGACAACGAAGG-3'
CD8.RO   :  5'-XGGATAATCGACTCACCC-3'
```

où X représente un colorant fluorescent (Fam) qui a été ajouté comme préconisé par le fournisseur (Applied Biosystems).

5) La préparation de l'ARN et de l'ADNc - les ARN soit d'organes, soit d'un nombre connu de cellules ont été purifiés sur gradients de chlorure de césium (20), puis transcrits par transcriptase inverse à l'aide du kit Boehringer pour la synthèse de l'ADNc. Le cas échéant, le rendement de la transcription inverse a été estimé par mesure de l'incorporation de $\alpha^{32}$P-dCTP.

6) L'amplification par PCR - Les réactions d'amplification ont été effectuées dans un mélange de 50 µl contenant 20U/ml de Taq polymérase (Promega), 200 µM de dNTP, 0,5 µM de chacune des deux amorces, 1,5 mM de Mg$^{++}$, dans du tampon Promega. Les mélanges réactionnels ont été recouverts de 50 µl d'huile minérale. Les amplifications ont commencé par une étape de dénaturation de 1 min à 94°C, suivie de cycles (40 habituellement) qui consistent chacun en 1 min à 94°C, 1 min à 60°C, 4 min à 72°C (pour minimiser la recombinaison intra-PCR (21)), et se sont terminées par une étape de 10 min à 72°C.

7) Réaction d'élongation supplémentaire ('run off') ou amplification complémentaire - Deux µl de la solution amplifiée ont été mélangés dans un volume réactionnel final de 10 µl contenant 0,1 µM de l'oligonucléotide marqué par un colorant, 20 U/ml de Taq polymérase, 200 µM de dNTP, 3 mM de Mg$^{++}$, dans du tampon Promega. L'élongation de l'amorce a commencé par une étape de dénaturation de 2 min à 94°C, suivie de 1 min à 60°C et s'est terminée par 15 min à 72°C.

8) Electrophorèse et analyse - les produits de la réaction d'amplification complémentaire ont été mélangés avec un volume égal d'une solution d'EDTA-formamide à 20mM, dénaturés par la chaleur à 80°C pendant 10 min et 2 μl du mélange résultant ont été déposés sur un gel d'acrylamide à 4% et à 8 M d'urée et soumis à électrophorèse pendant 4 h à l'aide d'un séquenceur d'ADN 373A Allied Biosystems. Un logiciel permet de mesurer, pour chaque pic d'ADN détecté, à la fois sa longueur (avec une précision inférieure à 0,2 nucléotides) et sa surface (avec une précision inférieure à 5%).

Lorsque le nombre de molécules d'ADNc simple brin a été mesuré à l'aide d'ADN étalon double brin, les valeurs expérimentales sont multipliées par deux.

## RESULTATS

### 1) Principes de la méthodes

a) Il s'agit de la co-amplification de l'échantillon d'intérêt avec un étalon interne d'ADN à l'aide de la même paire d'amorces, mais il est nécessaire que la divergence entre la séquence de l'ADN étalon et celle de l'ADN dosée soit aussi faible que possible (typiquement de quelques paires de base sur quelques centaines).
b) La lecture de la réaction d'amplification nécessite un ou deux oligonucléotdes supplémentaires qui permettent de faire la distinction entre les deux espèces d'ADN résultant de l'amplification de l'échantillon et l'ADN étalon respectivement.

Ces deux principes se justifient pour les raisons suivantes:

a) La quasi-identité entre l'étalon et l'ADN de l'échantillon assure leur amplification dans les mêmes conditions, même à la saturation, lorsque les molécules simple brin entrent en compétition les unes avec les autres au cours des dernières étapes de l'amplification. La co-amplification des deux séquences d'ADN présentant des grandes différences dans la région intra-amorces peut produire des déviations significatives en fin de la phase exponentielle (figure 9).
b) Lorsque les réactions d'amplification ont été conduites jusqu'au plateau, des séquences contaminantes sont parfois amplifiées. La lecture avec une (ou deux) amorce(s) supplémentaire(s) élimine un tel bruit de fond inutile et permet une double vérification de la spécificité. Cela est particulièrement important lorsque la méthode s'étend à la quantification simultanée de plusieurs gènes ou transcrits.

### 2) Protocole expérimental

Les deux principes précédents ont été mis en oeuvre en pratique de la manière suivante:
Premièrement, l'ADN étalon a été engendré en créant une petite insertion ou délétion (typiquement de 4 nucléotides sur quelques centaines) dans la séquence sauvage. Une manière adéquate a consisté à digérer l'ADN sauvage par une enzyme de restriction, créant des extrémités sortantes qui ont alors été coupées ou remplies par une polymérase et religuées ultérieurement (les plasmides utilisés comme étalons sont marqués ci-dessous par un astérisque).
Deuxièmement, on a détecté les produits de PCR en effectuant une étape d'amplification complémentaire déclenchée par une amorce supplémentaire marquée, insérée entre les amorces utilisées dans l'amplification, qui s'hybride aussi bien avec les deux espèces amplifiées. Dans la mesure où l'étalon et l'ADN de l'échantillon étaient de taille différente, il a été possible d'effectuer l'étape d'amplification complémentaire de telle sorte que la différence entre les deux produits d'élongation était de 4 nucléotides et ils pouvaient par conséquent être facilement séparés par électrophorèse. Ici, les produits sont déposés sur un gel de séquençage et analysés dans un séquenceur d'ADN automatique (ABI). La fluorescence a été enregistrée et les surfaces des pics mesurées. Le rapport des intensités des deux bandes a permis la quantification.
Dans les expériences décrites ci-dessous (sauf dans §3), on a effectué 40 cycles d'amplification et vérifié que les amplifications étaient bien menées à saturation.

### 3) Quantification de l'ADN

a) Validation des principes de la méthode. Pour valider l'utilité de l'approche, on a construit plusieurs plasmides dérivés de pH2 qui porte un fragment d'ADNc codant pour la molécule $K^d$ murine de la classe I d'histocompatibilité. Comme décrit dans Matériel et Méthodes et à la figure 8, ces trois plasmidescontenaient une séquence qui pouvait être amplifiée par la paire d'oligonucléotides H2.5' et H2.3'. Une troisième amorce H2.RO pouvait détecter les différents fragments. Ces produits de PCR contenaient le séquence $K^d$ murine de la classe I pour pH2, la même séquence contenant une petite insertion de quatre bases pour pH2*, et une séquence différente non utile de même longueur pour pH2-diff. La quantification d'un volume constant de l'échantillon de pH2 contenant environ $10^4$ copies a été effectuée en utilisant des quantités croissantes soit de pH2* soit de pH2-diff. Les résultats de la figure 9 montrent que la quantité, supposée inconnue, de pH2 dans l'échantillon à doser pouvait être mesurée de manière précise par rapport à l'échelle de pH2*. Les résultats sont très reproductibles comme on peut le constater à partir des écarts types présentés dans la figure 9 et ils sont clairement indépendants du nombre de cycles d'amplification réalisés. La linéarité est maintenue sur au moins 4 ordres de grandeur dans la mesure où des résultats analogues ont été obtenus avec des échantillons contenant à peine $10^2$ ou jusqu'à $10^6$ molécules de pH2, à condition que l'on ait fait varier l'échelle de pH2* de façon appropriée. De manière générale, 3 (ou même 2) concentrations de pH2* sont suffisantes pour construire une échelle de référence fiable à condition que la concentration approximative de pH2 soit connue à 2 ordres de grandeur près. La concentration de pH2 a alors été mesurée avec une précision estimée à environ ± 25%. Par contre, la quantification est impossible lorsque pH2-diff est utilisé comme molécule étalon dans la mesure où le rapport entre les deux espèces amplifiées ne traduit pas le rapport initial après un nombre de cycles saturants (figure 9). Lorsque la réaction atteint le plateau, les deux séquences se comportent de manière indépendante et sont donc amplifiées en tant que deux espèces différentes.

b) Sensibilité. L'efficacité du procédé a encore été illustrée par une exprérience de reconstitution dans laquelle de l'ADN d'HIV cloné, dilué dans de l'ADN humain, a été mélangé avec différentes quantités d'étalon interne pHIV* (cf. Matériel et Méthodes). 10, $10^3$ ou $10^6$ copies de pHIV, mélangées avec 1 µg d'ADN humain, correspond à $10^5$ cellules, ont été soumises à 40 cycles d'amplification avec les amorces HIV.5' et HIV.3', mélangées avec des copies de pHIV* sur une échelle appropriée. Les résultats expérimentaux sont très comparables aux dilutions connues d'ADN de l'HIV dans de l'ADN humain (données non représentées).

c) Adaptabilité. Plusieurs cycles de réactions d'amplification complémentaires peuvent également répondre aux deux principes initiaux de la méthode (c'est-à-dire quasi-identité des ADN à transcrire et forte spécificité), tout en augmentant linéairement le signal fluorescent en fonction du nombre de cycles. Cela est en effet le cas jusqu'à au moins 10 cycles (données non représentées). Ce procédé peut, par conséquent, être éventuellement utilisé pour accroître la sensibilité de la lecture.

### 3) Quantification de l'ARNm

a) Normalisation. Avec cette stratégie, la quantification de l'ARNm nécessite que celui-ci soit d'abord transcrit en ADNc par la transcriptase inverse. La méthode peut être utilisée sur l'ADNc dans exactement les mêmes conditions. Toutefois, l'extraction de l'ARN et la transcription inverse ont souvent des rendements plutôt variables (plus variables que l'extraction d'ADN). On a par conséquent mis au point des dosages pour les transcrits des gènes de ménage de façon à normaliser les résultats obtenus avec différents échantillons d'ARN et d'ADNc, en partant de la présupposition que ces gènes étaient transcrits dans les divers échantillons de manière équivalente. Deux ADN étalons ont été préparés, chacun étant 4 nucléotides plus court que les ARNm correspondants, un codant pour l'hypoxanthine phosphoribosyl-transférase (HPRT) de souris. Le gène HPRT, comme d'autres gènes de ménage, est exprimé à de très faibles niveaux dans la plupart des types cellulaires (22). Pour vérifier si ces gènes peuvent être utilisés comme témoin interne pour la quantité d' ARNm dans les expériences de quantification, on a quantifié le nombre de copies d'HPRT dans différents ADNc préparés à partir d'un nombre connu de cellules originaires de trois lignées cellulaires distinctes ainsi que de la rate de souris. Les résultats de ces quantifications sont présentés dans le tableau 1. On a d'abord mesuré le nombre de copies d'ADNc codant pour l'HPRT présent parmi l'ADNc résultant de la transcription inverse de 2 ng d'ARN cellulaire total. On a constaté que le nombre de copies variait de 900 à 4400 dans les différentes lignées cellulaires et tissus testés. En prenant en compte le rendement d'extraction d'ARN et le rendement de la transcription inverse, nous avons déduit que le nombre d'ARNm d'HPRT par cellule variait de 5 à 10 dans les trois lignées cellulaires testées. Dans toutes les expériences ultérieures, on a considéré que le nombre de copies d'ARNm d'HPRT par cellule était de 10.

b) Validation. On a alors quantifié la quantité d'ARNm de l'H-2K$^d$ (codant pour l'antigène majeur d'histocompatibilité classe I bien caractérisé, qui peut être détecté à la surface de la plupart des cellules de la souris BALB/c) présente dans les différents types cellulaires (H-2$^d$) et/ou organes, à l'aide de pH2*. L'ARN a été extrait des lignées cellulaires AC38, 42F8, X63Ag8.IL2 et des tissus de souris BALB/c, et transcrit en ADNc par transcriptase inverse. Des aliquots ont été mélangés avec pH2* ou pHPRT* (dans des expériences séparées) pour l'amplification et l'amplification complémentaire. On a constaté que: 1) le combre de copies d'ADNc d'H-2K$^d$ présent dans l'ADNc obtenu de la transcription inverse de 2ng d'ARN total variait entre 90 (pour X63Ag8.IL2) et 57000 (pour le poumon de BALB/c). 2) après renormalisation par rapport au niveau d'ADNc d'HPRT, le nombre de copies d'ADNc d'H-2K$^d$ détecté pour 10 copies d'ADNc d'HPRT (c'est-à-dire par cellule) variait entre 0.8 (pour X63Ag8.IL2) et 290 (pour le poumon de BALB/c). Le nombre de copies détecté dans les organes était en corrélation avec les valeurs attendues: tout d'abord, il est connu que l'ARNm d'H-2K représente environ 0.05% de tous les ARNm dans le foie de souris (16). Comme il existe environ 100.000 molécules d'ARNm par cellule, le nombre de copies d'ARNm d'H-2K$^d$ par cellule doit être de l'ordre de 50 dans un organe tel que le foie. Le thymus et le poumon expriment les molécules de la classe I à un taux plus élevé et par contre, il est connu que le cerveau exprime l'H-2K à un taux très faible (23), ce qui est en accord avec les résultats.

De plus, on a ensuite établi la corrélation entre le niveau d'expression de l'ARNm codant pour H2-K$^d$ dans deux de ces lignées cellulaires et le niveau K$^d$ exprimé à leur surface. Les expériences cytométriques montrent que AC38 exprime K$^d$ 1,7 fois plus que X63Ag8.IL2, ce qui est très comparable au rapport du taux d'expression d'ARNm que l'on trouve dans ces deux lignées cellulaires (rapport de 1,9; voir tableau 1).

c) Application. Enfin, on a étudié la réponse immunitaire contre la cytochrome C de pigeon (PCC) chez la souris B10.A. La réponse des cellules T contre le PCC a été abondamment caractérisée (24, 25) et il est connu que la réponse est dominée par l'apparition d'un clone de cellule T dont la chaîne β des récepteurs des cellules T utilise les segments Vβ3 et Jβ1.2 avec une région codant pour CDR3 de 27 paires de base (18, 26). Grâce à cette information, aux oligonucléotides spécifiques précédemment décrits (Vβ3.A', Jβ1.2.3 et Vβ3.B: RO) et un ADN étalon pVβ3*, on a pu quantifier cet ARNm de la chaîne β des récepteurs des cellules T en même temps que l'ARNm d'HPRT au cours de l'immunisation des animaux. Après normalisation par rapport à l'expression de l'ARNm d'HPRT, il est évident que le nombre de copies d'ARNm est augmenté de 2 (après deux semaines) et de 3 (après restimulation in vivo au jour 49) ordres de grandeur par rapport à ceux des souris non immunisées (figure 10).

**4) Quantification de plusieurs sequences d'ADN ou d'ARNm.** Il est possible de construire un système de détection dans lequel des séquences géniques et les ADN étalons correspondants sont organisés d'une telle manière que plusieurs paramètres peuvent être quantifiés dans une seule étape d'électrophorèse. Cela nécessite simplement que les amorces pour la PCR et pour l'élongation complémentaire soient choisies de manière à engendrer des produits d'élongation de tailles adéquates. Pour illustrer cette approche, on a quantifié les ARNm codants pour les protéines de surface CD4 et CD8, qui sont spécifiques de sous ensembles bien caractérisés de cellules T, de la manière suivante; les produits de l'élongation complémentaire sont de 96 nucléotides pour pCD4*, de 100 pour l'ARNm de CD4, 106 pour pCD8* et 110 pour l'ARNm de CD8. Les résultats de la figure 11 montrent que les deux ARNm quantifiés séparément à partir du même échantillon d'ARN, peuvent être analysés conjointement. Ils démontrent également que les différences dans l'efficacité des différentes paires d'oligonucléotides mises en oeuvre pour ces amplifications, conduisant parfois à des variations de l'intensité globale d'un produit de co-amplification, ne constituent pas un obstacle à cette technique.

TABLEAU 1

| | Nombre de copies d'ADNc d'HPRT | Nombre de copies d'ARNm d'HPRT par cellule | Nombre de copies d'ADNc de Kd | Nombre de copies d'ADNC de Kd pour 10 copies d'ADNc d'HPRT |
|---|---|---|---|---|
| **Lignées cellulaires:** | | | | |
| AC38 | 1768 | 10 | 264 | 1,5 |
| 42F8 | 912 | 5 | 1001 | 11,0 |
| X63Ag8.I12 | 1110 | 9 | 91 | 0,8 |
| **Organes:** | | | | |
| Poumon | 1956 | - | 56767 | 290,2 |
| Thymus | 2897 | - | 20904 | 72,2 |
| Cerveau | 4380 | - | 3740 | 8,5 |

TABLEAU 1: Quantification du nombre de copies d'ARNm codant pour H-2K$^d$ ou HPRT dans différentes lignées cellulaires et organes de souris. Le nombre de copies d'ADNc codant soit pour H-2K$^d$ soit pour HPRT présent dans l'ADNc résultant de la transcription inverse de 2 ng d'ARN total a été déterminé dans différentes lignées cellulaires ou organes. Les différents ADNc ont été amplifiés dans des expériences différentes avec les séries de dilutions appropriées de l'ADN étalon adéquat. Le nombre de copies de l'ARNm d'HPRT par cellule a ensuite été calculé en supposant que le rendement de synthèse de l'ADNc était égal à 1.

## DISCUSSION GENERALE

On a décrit ici un protocole général qui permet de quantifier les réactions d'amplification enzymatiques - et plus particulièrement celle la plus utilisée, la PCR menée à saturation. Il est important en pratique que de telles réactions soient menées à saturation pour au moins trois raisons convergentes: simplicité opérationnelle (l'arrêt des réaction dans leur phase exponentielle nécessite des opérations complexes, telles que de s'assurer que le plateau n'était en effet pas déjà atteint au moment de l'arrêt de l'amplification), sensibilité (davantage de matériel à doser), et reproductibilité (le nombre de cycles nécessaires pour atteindre le plateau est très variable d'un échantillon à l'autre et même d'une expérience a l'autre). En d'autres termes, le problème majeur de la PCR quantitative est de dissocier la quantification du rendement. L'approche pour résoudre ce problème est ici basée sur deux principes, à savoir: (a) que l'étalon interne mis en oeuvre pour la co-amplification est aussi similaire que possible a la séquence à doser, et (b) que un ou deux oligonucléotides supplémentaires sont mis en oeuvre pour la lecture de la réaction d'amplification.

Ce premier principe garantit que l'ADN étalon et l'échantillon sont amplifiés en tant que population unique dans toutes les circonstances, quelque soit le nombre de cycles, et, en grande partie, l'efficacité intrinséque des amorces oligonucléiques. Le deuxième prévoit une double vérification de la spécificité de la réaciton d'amplification (les contaminants présents occasionnellement ne seront pas reconnus par l'amorce (les amorces) supplémentaires(s)) ainsi qu'un moyen de faire la distinction entre l'ADN étalon et l'ADN à doser.

Dans cet exemple, on s'est servi de la différence introduite dans la taille de l'ADN étalon pour faire la distinction entre les deux espèces amplifiées en réalisant une réaction d'amplification complémentaire déclenchée par l'introduction d'un oligonucléotide fluorescent supplémentaire. Toutefois, comme il est discuté dans ce qui suit, d'autres protocoles expérimentaux peuvent être mis en oeuvre.

Les résultats exprérimentaux montrent que si les deux principes de départ sont satisfaits, on peut quantifier de manière reproductible l'ADN ou l'ARNm par PCR (ou d'autres réactions d'amplification) menée à saturation, avec une précision estimée à ± 25%. Ils montrent également que

(a) l'utilisation d'étalons internes d'ADN dont la séquence est aussi voisine que possible de l'échantillon à quantifier est une condition majeure, à moins que les distorsions parfois provoquées par les différences de séquences ne soient corrigées. Si toutefois, comme le suggèrent les résultats, ces distorsions se manifestent au niveau du plateau d'une manière quelque peu aléatoire, les corrections ne seront pas fiables.

(b) l'utilisation d'amorces supplémentaires pour déclencher les réactions d'amplification complémentaires, comme indiqué dans notre deuxième principe, n'est pas une exigence absolue: si les amorces mises en oeuvre pour l'amplification sont très spécifiques, il y aura peu de bruits de fond non spécifiques au niveau du plateau. Si l'ADN étalon et l'échantillon satisfont au premier principe et sont de tailles différentes, on peut les séparer directement par électrophorèse. De plus, le calibrage précis réalisé dans des gels de séquençage représente en soi une double vérification de la spécificité car il est peu probable - bien que pas rigoureusement exclu - que les espèces d'ADN contaminants aient la même taille que celle de l'échantillon et/ou l'ADN étalon, Toutefois, l'utilisation d'oligonucléotides supplémentaires est intéressante pour deux raisons: tout d'abord, la double vérification de la spécificité est plus rigoureuse et améliore la fiabilité de la méthode. Deuxièmement, si plusieurs échantillons sont mélangés et analysés dans la même colonne d'un gel d'électrophorèse, il devient possible, voire probable, que la taille des espèces contaminantes engendrées dans l'une ou l'autre réaction d'amplification soit du même ordre que celle des échantillons ou des étalons à mesurer. Ceci a été observé en amplifiant un ADNc directement avec les amorces CD4.5' et CD4.RO ou CD8.5' et CD8.RO. Lorsque les produits de PCR ont été déposés et analysés sur un gel, il est apparu que l'amplification avec les amorces CD8 engendrait des produits non spécifiques de 90 nucléotides de long, ce qui correspond à la taille du produit spécifique avec les amorces CD4 (résultat non représenté). Ainsi, une des conditions pour le mélange d'échantillons en vue de leur analyse est l'absence totale de bruit de fond, ce qui est mieux assuré par l'utilisation d'amorces supplémentaires et de réactions d'amplification complémentaire.

Toutefois, l'utilisation de paires d'oligonucléotides supplémentaires spécifiques à l'échantillon et à l'ADN étalon ouvre la voie à des méthodes de détection plus simples, qui ne font pas appel à l'électrophorèse. Cette caractéristique est intéressante pour les tests de routine futurs faisant appel à la PCR ou à d'autres méthodes d'amplification.

Plusieurs protocoles ont été décrits pour la mesure rapide et quantitative des produits de PCR. Parmi ceux-ci, la possibilité de réaliser la lecture de la réaction avec deux oligonucléotides chevauchant les quelques nucléotides qui différencient l'ADN à doser, est particulièrement intéressante. De tels procédés sont adaptés pour une lecture enzymatique directe. Ils peuvent rendre l'analyse de la lecture à la fois plus courte et plus simple.

Grâce au procédé de l'invention, on peut quantifier de l'ADN et de l'ARNm, ce dernier étant d'abord transcrit en ADNc par la transcriptase inverse. Parmi plusieurs exemples, on a mesuré la quantité d'ARNm codant pour un antigène

**EP 0 613 503 B1**

de souris majeur ($K^d$) de transplantation de classe I, dans plusieurs lignées cellulaires et on a observé une bonne corrélation avec son expression à la surface, évaluée par des mesures de FACS. Pour ces mesures d'ARNm, on a jugé utile d'incorporer un témoin interne supplémentaire, savoir la quantification des ARNm codant pour des protéines de ménage (HPRT ou β-actine) de manière à normaliser l'extraction d'ARN et les rendements en transcription inverse, Enfin, il a été possible, pour des ARN distincts et leurs étalons internes, d'utiliser des amorces donnant des produits de l'amplification complémentaire avec une hiérarchie de tailles connue. A titre d'exemple, on montre que les ARNm codant pour les marqueurs de surface des cellules T, CD4 et CD8, peuvent être quantifiés en une seule étape d'électrophorèse. On choisit des intervalles tels qu'un couple de produits de l'amplification complémentaire correspondant aux ARNm et leurs étalons occupe 10 nucléotides. Par conséquent, au moins 25 couples peuvent en principe être mélangés et soumis à l'électrophorèse dans une colonne du séquenceur automatique. Etant donné que marqueurs fluorescents et 36 colonnes peuvent être utilisées, il est possible d'analyser 3600 couples simultanément. En supposant que le dosage d'un ADN ou ARNm nécessite l'analyse de 3 couples (avec des concentrations variables d'ARN étalon), quelque 1200 échantillons peuvent être quantifiés en une seule opération. Même si ce chiffre est en excès en raison de la nécessité d'incorporer plusieurs témoins, le potentiel analytique est considérable. On peut en tout cas réaliser un kit pour permettre la quantification de 25 ARNm distincts dans un seul échantillon.

En résumé, le procédé de l'invention permet de mesurer, avec une précision d'environ ± 25%, la(les) quantité(s) d'une ou de plusieurs séquence(s) d'ADN et/ou d'ARNm présente(s) dans un échantillon biologique, à l'aide de réactions d'amplification telles que la PCR menée à saturation. De telles méthodes s'avèrent extrêmement utiles dans des situations très diverses. On a mis en évidence ici que les séquences d'HIV peuvent être dosées ainsi de manière quantitative et avec une grande sensibilité. Pour la première fois, l'apparition d'un clone spécifique de cellules T murines a pu être quantifiée au cours d'une réponse immunitaire. Les marqueurs des cellules T murines, CD4 et CD8 ont pu être facilement dosés et analysés simultanément. Les deux derniers essais peuvent être utiles pour l'analyse de diverses situations pathologiques chez l'homme (telles que la nature des lymphocytes T s'infiltrant dans une tumeur ou un organe soumis à une attaque auto-immune). Une grande variété de tests peuvent être développés conformément à ce procédé.

## <u>REFERENCES</u>

1 - Saiki, R.K., Scharf, S., Faloona, Mullis, K.B., Horn., G.T., Erlich. H.A. and Arnheim, N. (1985), Science 230, 1350-1354.

2 - Erlich, H.A., Gelfand, D. and Sninsky, J.J. (1991), Science 252, 1643-1651.

3 - Walker, G.T., Fraiser, M.S., Schram, J.L., Little, M.C., Nadeau, J.G. and Malinowski, D.P. (1992). Nucl. Acids Res. 20, 1691 - 1696.

4 - Compton, J. (1991), Nature 350, 91-92.

5 - Kwoh, D.Y., Davis, G.R., Whitfield, K.M., Chapelle, H. L., DiMichele, L.J. and Gingeras, T.R. (1989), Proc. Natl. Acad. Sci. USA 86, 1173-1177.

6 - Guatelli, J.C., Whitfield, K.M., Kwoh, D.Y., Barringer, K.J., Richman, D.D. and Gingeras, T.R. 1990). Proc. Natl. Acad. Sci. USA 87, 1874-1878.

7 - Chelly, J., Montarras, D. Pinset, C., Berwald-Netter, Y., Kaplan, J.C. and Kahn, A. (1990), Eur. J. Biochem. 187, 691-698.

8 - Hoof, T. Riordan, J.R. and Tummler, B. (1991). Anal. Biochem. 196, 161-169.

9 - Ballagi, P.A., Ballagi, P.A. and Funa, K. (1991). Anal. Biochem. 196, 89-94.

10 - Robinson, M.O. and Simon, M.I. (1991). Nucl. Acids Res. 19, 1557-1562.

11 - Wang, A.M., Doyle, M.V. and Mark, D.F. (1989). Proc. Natl. Acad. Sci. USA 86, 9717-9721.

12 - Gilliland, G., Perrin, S., Blanchard, K. and Bunn, H.F. (1990). Proc. Natl. Acad. Sci. USA 87, 2725-2729.

13 - Reth, M. (1982). Thesis, Université de Cologne.

14 - Karasuyama, H. and Melchers, F. (1988). Eur. J. Immunol. 18, 97-104.

15 - Rebai, N., Mercier, P., Kriestensen, T. Devaux, C., Malissen, B. Mawas C. and Pierres, M. (1983). Immunogenetics 17, 353-370.

16 - Lalanne, J.-L., Delarbre, C., Gachelin, G. and Kourilsky, P. (1983). Nucl. Acids Res. 11, 1567-1577.

17 - Meyerhans, A. Cheynier. R. Albert. J., Seth, M., Kwoh, S., Sninsky, J., Morfeldt-Manson, L., Asjö, B. and War,- Hobson, S. (1989), Cell 58, 901-910.

18 - Fink, P.J., Matis, L.A., McElligott, D.L., Bookman, M. and Hedrick, S.M. (1986). Nature 321, 219-226.

19 - Sanger, F., Nicklen, S. and Coulson, A.R. (1977), Proc. Natl. Acad. Sci. USA 74, 5463-5467.

20 - Chirgwin, J.M., Przybyla, A.E., MacDonald, R.J. and Rutter, W.J. (1979). Biochemistry 18, 5294-5299.

21 - Meyerhans, A., Vartanian, J.-P. and Wain-Hobson, S. (1990). Nucl. Acids Res. 18, 1687-1691.

22 - Melton, D.W., Konecki, D.S., Brennand, J. and Caskey, C.T. (1984). Proc. Natl. Acad. Sci. USA 81, 2147-2151.

23 - David-Watine, B., Israël, A. and Kourilsky, P. (1990). Immunol. Today 11, 286-292.

24 - Hedrick, S.M. (1988). Adv. in Immunol. 3, 193.

25 - Fink, P.J., Blair, M.J., Matis, L.A. and Hedrick, S.M. (1990). J. Exp. Med. 172, 139-150.

26 - Sorger, S.B., Hedrick, S.M., Fink, P.J., Bookman, M.A. and Matis, L.A. (1987). J. Exp. Med. 165, 279-301.

27 - BARTH, K., Kim, B.S., Lan, N.C., Hunkapiller, T., Sobieck, N. Winoto, A., Gershenfeld, H., Okada, C., Hansburg, D. Weissman, I.L., and Hood. L. (1985). The murine T-cell receptor uses a limited repertoire of expressed Vbeta gene segments. Nature 316, 517-523.

28 - Dower, W.J., Miller, J.F., and Ragsdale, C. (1988). High efficiency transformation of E. Coli by high voltage electroporation. Nucl. Acids. Res. 16, 6127-6145.

29 - Kellog, D.E., Sninsky, J.J., and Kwok S. (1990). Quantification of HIV-1 proviral DNA relative to cellular DNA by the polymerase chain reaction. Anal. Biochem 189, 202-208. Mullis, K.B. and Faloona. F. (1987). Specific synthesis of DNA in vitro via a polymerase-catalyzed chain reaction. Methods Enzymol. 155? 335-350.

30 - Maire, P., Gautron, S., Hakim V., Gregori, C., Mennecier, F., and Kahn, A. (1987). Characterization of three optional promoters in the 5' region of the human aldolase A gene. J. Mol. Biol. 197, 425-438.

31 - Mestek, A., Stauffer, J., Tolan, D.R., and Ciejek-Baez, E. (1987). Sequence of a mouse brain aldolase A cDNA. Nucl. Acids Res. 15, 10595.

32 - Singer-Sam, J., Robinson, M.O., Bellivé, A.R., Simon, M.I., and Riggs, A.D. (1990). Measurement by quantitative PCR of changes in MPRT, PGK-1, PGK-2, APRT, MTase, and Zfy gene transcripts during mouse spermatogenesis. Nucleic Acids Res. 18, 1255-1259.

33 - Van den Eynde, B., Lethé, B., Van Pel, A., DXe Plaen, E., and Boon, T. (1991). The gene coding for a major tumor rejection antigen of tumor P815 is identical to the normal gene of syngeneic DBA/2 mice. J. Exp. Med. 173, 1373-1384.

34 - Wang, A. M. Doyle, M.V., and Mark, D.F. (1989). Quantification of mRNA by the polymerase chain reaction. Proc. Natl. Acad. Sci. 86, 9711-9721.

35 - Woodcock, D.M., Crawther, P.J., Doherty, J. Jefferson, S., Debruz, E., Noyer-Weidner, M. Smith, S.A., Michael, M.Z., and Graham M.W. (1989) - Quantitative evolution of Escherichia Coli host strains for tolerance to cytosine methylation in plasmid and phage recombinants. Nucl. Acids Res. 17, 3469-3478.

## Revendications

1. Procédé de détermination de la quantité d'un fragment d'ADN d'intérêt dans un échantillon à analyser par une méthode d'amplification enzymatique in vitro dudit fragment, caractérisé en ce que :

   1) on ajoute à l'échantillon à analyser contenant le fragment d'ADN d'intérêt, un fragment d'ADN standard différent du fragment d'ADN d'intérêt mais amplifiable par les mêmes oligonucléotides amorces, les fragments d'ADN standards et d'intérêt ne différant en séquences et/ou en taille de pas plus de 10% environ, de préférence de pas plus de 5 nucléotides par brin,

   2) on co-amplifie les fragments d'ADN intérêt et standard avec les mêmes oligonucléotides amorces, de préférence jusqu'à saturation de l'amplification du fragment d'ADN d'intérêt,

   3) on ajoute dans le milieu réactionnel obtenu à l'étape 2) :

   - soit deux types d'oligonucléotides marqués sondes spécifiques, chacun respectivement des fragments d'ADN d'intérêt et standards et différents des oligonucléotides amorces d'amplification de l'étape 2),
   - soit un ou plusieurs oligonucléotide(s) amorce(s) marque(s), spécifique(s) aux fragments d'ADN d'intérêt et standards, et différents desdits oligonucléotides amorces de l'étape 2), et on effectue un ou quelques cycle(s) d'amplification supplémentaire(s) avec le ou lesdits oligonucléotide(s) amorce(s) marqué(s), de sorte que, au cours d'un cycle, après dénaturation de l'ADN, le ou lesdits oligonucléotide(s) amorce(s) marqué(s) s'hybride(nt) avec lesdits fragments en un site approprié pour qu'une élongation par l'ADN polymérase génère des fragments d'ADN marqués de tailles et/ou de séquences différentes ou avec des marqueurs différents selon qu'ils proviennent des fragments d'ADN d'intérêt ou standards respectivement, puis

   4) on détermine la quantité initiale de fragment d'ADN d'intérêt comme étant le produit de la quantité initiale de fragment d'ADN standard et du rapport entre la quantité de fragment d'ADN d'intérêt amplifié et la quantité de fragment d'ADN standard amplifié, rapport qui est identique à celui des quantités des fragments d'ADN marqués provenant respectivement des fragments d'ADN d'intérêt et standards amplifiés obtenus à l'étape 3).

2. Procédé selon la revendication 1, caractérisé en ce que les fragments d'ADN standard et d'ADN d'intérêt différent en au moins un site repérable par quelque moyen que ce soit.

3.  Procédé selon la revendication 1 ou 2, caractérisé en ce que les tailles des fragments d'ADN d'intérêt et standards diffèrent d'au moins 1 et d'au plus 10% de nucléotides ou de 1 à 5 nucléotides par brin.

4.  Procédé de détermination de la quantité d'un fragment d'ADN d'intérêt par une méthode d'amplification enzymatique in vitro d'ADN selon l'une des revendications 1 à 3, caractérisé en ce que, à l'étape 3), on ajoute dans le milieu réactionnel obtenu à l'étape 2) un troisième oligonucléotide amorce marqué, amorce commune aux fragments d'ADN d'intérêt et standards, et on effectue un ou quelques cycle(s) d'amplification supplémentaire avec ladite troisième amorce, tel que, au cours d'un cycle, après dénaturation de l'ADN, ladite troisième amorce s'hybride spécifiquement avec lesdits fragments en un site approprié pour qu'une élongation par l'ADN polymérase génère deux types de fragments d'ADN marqués de tailles différentes selon qu'ils proviennent des fragments d'ADN d'intérêt et standards respectivement et comportant chacun une partie seulement de ces derniers respectivement.

5.  Procédé selon l'une des revendications 1 à 4 caractérisé en ce que le fragment d'ADN standard correspond au fragment d'ADN d'interêt dans lequel on a effectué une ou des délétion(s), mutation(s) et/ou addition(s) de nucléotides.

6.  Procédé selon l'une des revendications 4 ou 5, caractérisé en ce que le fragment d'ADN standard correspond au fragment d'ADN d'intérêt dans lequel on a effectué une délétion, mutation ou addition en un site de 1 à 5 nucléotides, de préférence 3 à 4, et en ce que ladite troisième amorce est un oligonucléotide complémentaire d'une séquence située en amont de ladite délétion, mutation ou addition.

7.  Procédé de détermination de la quantité d'un ADN d'interêt selon l'une des revendications 1 à 6, caractérisé en ce que:

    a) les fragments d'ADN standards et d'intérêt ont la même taille et diffèrent en séquence par un site de restriction d'une endonucléase particulière ou tout autre site de coupure réalisable par tout autre moyen, et
    b) à l'étape 3), on ajoute au milieu réactionnel obtenu à l'étape 2) un troisième oligonucléotide amorce marqué, amorce commune aux fragments d'ADN d'intérêt et standards, et on effectue un ou plusieurs cycle(s) d'amplification supplémentaire(s) avec ladite troisième amorce, de sorte, qu'au cours d'un cycle, après dénaturation de l'ADN, ladite troisième amorce s'hybride spécifiquement avec lesdits fragments en un site approprié pour qu'une élongation par l'ADN polymérase génère deux types de fragments d'ADN marqués se distinguant selon qu'ils proviennent des fragments d'ADN d'intérêt et standards respectivement, en ce que l'un desdits fragments comporte toujours ledit site de restriction et
    c) avant l'étape 4), on effectue une digestion des produits d'amplification de l'étape 3), à l'aide de ladite endonucléase, ou tout autre moyen de coupure correspondant au site de coupure, de manière à générer des fragments de tailles différentes selon qu'ils proviennent des fragments d'ADN d'intérêt ou standards.

8.  Procédé selon l'une des revendications 1 à 7, caractérisé en ce que la détermination de l'étape 4) se fait en

    -  séparant selon leur taille par électrophorèse sur gel les fragments d'ADN marqués provenant des fragments d'ADN d'intérêt et standards amplifiés, puis en
    -  détectant les intensités des signaux correspondant au marqueur de la ou desdites amorce(s) respective(s) pour les fragments d'ADN marqués provenant des fragments d'ADN d'intérêt et standards respectivement.

9.  Méthode selon l'une des revendications 1 à 7 caractérisée en ce qu'on sépare les fragments d'ADN amplifiés marqués provenant des fragments d'ADN d'intérêt et standards amplifiés selon leur taille, à l'aide d'un dispositif d'électrophorèse sur gel avec séquençage d'ADN.

10. Procédé selon l'une des revendications précédentes d'analyse multiparamétrique permettant de doser et quantifier plusieurs fragments d'ADN d'intérêt dans un même échantillon analysé, caractérisé en ce que :

    -  on ajoute à l'échantillon à analyser, contenant lesdits fragments d'ADN d'intérêt, autant de fragments d'ADN standards, les fragments d'ADN standard étant différents pour chacun des fragments d'ADN d'intérêt, et
    -  on choisit les ADN standards et les oligonucléotides marqués de l'étape 3), de façon à générer, au cours de l'étape 3), des fragments de tailles différentes.

**11.** Procédé de détermination de la quantité d'un fragment d'ADN d'intérêt selon l'une des revendications 1 à 5, par une méthode d'amplification enzymatique in vitro caractérisé en ce que à l'étape 3), on ajoute dans du milieu réactionnel obtenu à l'étape 2) deux oligonucléotides, sondes ou amorces différents des amorces d'amplification de l'étape 2), spécifiques des fragments d'ADN d'intérêt et standards respectivement et marqués par des marqueurs différents ; puis, le cas échéant, on effectue un ou quelques cycle(s) d'amplification supplémentaire(s) avec lesdits deux oligonucléotides amorces marqués, de sorte, qu'au cours d'un cycle, après dénaturation de l'ADN et élongation par une ADN polymérase, les deux dits oligonucléotides marqués génèrent deux types de fragments d'ADN marqués par des marqueurs différents selon qu'ils proviennent d'ADN d'intérêt ou standards.

**12.** Procédé selon la revendication 11, caractérisé en ce que :

a) à l'étape 2), les amorces d'amplification sont modifiées chimiquement de manière à pouvoir être liées à une phase solide, et
b) après l'étape 3), on lie les produits d'amplification à la phase solide par l'intermédiaire desdites amorces modifiées, puis la phase solide est lavée de manière à éliminer les amorces marquées en excès de l'étape 3) n'ayant pas réagi,

**13.** Procédé selon la revendication 11 ou 12, caractérisé en ce que si les fragments d'ADN marqué d'intérêt et standard ne different qu'en un seul site, les deux oligonucléotides amorces de l'étape 3) incluent ce site.

**14.** Procedé selon l'une des revendications 11 à 13, caractérisé en ce que les amorces d'amplification à l'étape 2) sont biotinylées et peuvent réagir et se lier avec la streptavidine laquelle est couplée à la phase solide.

**15.** Procédé selon l'une des revendications 1 à 14 caractérisée en ce qu'on réitère plusieurs fois le procédé dans les mêmes conditions mais en partant avec des quantités de fragments d'ADN standards initiales différentes.

**16.** Procédé selon l'une des revendications 1 à 15 caractérisé en ce que la ou lesdites amorce(s) est(sont) marquée (s) par un marqueur fluorescent.

**17.** Procédé selon l'une des revendications 1 à 16 caractérisé en ce que la méthode d'amplification enzymatique est la méthode PCR.

**18.** Kit de détermination de la quantité d'un fragment d'ADN d'intérêt par un procédé selon l'une des revendications 1 à 16, caractérisé en ce qu'il comporte :

- un fragment d'ADN standard ne différant en taille et/ou en séquence de pas plus de 10% environ, de préférence de pas plus de 5 nucléotides par brin du fragment d'ADN d'intérêt,
- les réactifs pour la mise en oeuvre de ladite méthode d'amplification enzymatique in vitro et notamment des oligonucléotides amorces,
- un ou plusieurs oligonucléotide(s), sondes ou amorce(s) marqué(s) qui s'hybride(nt) spécifiquement avec les fragments d'ADN standard et d'intérêt, le cas échéant, à un site approprié pour qu'une élongation par l'ADN polymérase génère des fragments d'ADN marqués de tailles et/ou de séquences différentes ou avec des marqueurs différents selon qu'ils proviennent des fragments d'ADN d'intérêt et standards respectivement.

**Patentansprüche**

**1.** Verfahren zur Bestimmung der Menge eines interessierenden DNA-Fragments in einer zu analysierenden Probe durch enzymatische Amplifikation des genannten Fragments in vitro, dadurch gekennzeichnet, daß man

1) der zu analysierenden Probe, die das interessierende DNA-Fragment enthält, ein Standard-DNA-Fragment zugibt, das von dem interessierenden DNA-Fragment verschieden ist, das jedoch durch die gleichen Starter-Oligonucleotide amplifizierbar ist, wobei sich die Standard- und interessierenden DNA-Fragmente in den Sequenzen und/oder in der Größe um nicht mehr als etwa 10 %, vorzugsweise um nicht mehr als 5 Nucleotide pro Strang, voneinander unterscheiden,
2) die interessierenden und Standard-DNA-Fragmente mit den gleichen Starter-Oligonucleotiden coamplifiziert, vorzugsweise bis zur Sättigung der Amplifikation des interessierenden DNA-Fragments,
3) dem in der Stufe (2) erhaltenen Reaktionsmedium zugibt:

- entweder zwei Typen von markierten Oligonucleotid-Sonden, die spezifisch sind jeweils für die interessierenden und die Standard-DNA-Fragmente und verschieden sind von den Amplifikations-Starter-Oligonucleotiden der Stufe (2),

- oder ein oder mehrere markierte Starter-Oligonucleotide, die spezifisch sind für die interessierenden und die Standard-DNA-Fragmente und verschieden sind von den genannten Starter-Oligonucleotiden der Stufe (2), und daß man einen oder mehrere zusätzliche Amplifikations-Cyclen mit dem oder den genannten markierten Starter-Oligonucleotiden in der Weise durchführt, daß im Verlaufe eines Cyclus nach der Denaturierung der DNA das oder die genannten markierten Starter-Oligonucleotide sich hybridisiert (hybridisieren) mit den genannten Fragmenten an einer geeigneten Stelle, so daß eine Verlängerung durch die DNA-Polymerase markierte DNA-Fragmente mit unterschiedlichen Größen und/oder Sequenzen ergibt, oder mit Markern, die verschieden sind, je nachdem, ob sie aus interessierenden bzw. Standard-DNA-Fragmenten stammen, und dann

4) die Anfangsmenge des interessierenden DNA-Fragments bestimmt als das Produkt der Anfangsmenge des Standard-DNA-Fragments und des Verhältnisses zwischen der Menge des amplifizierten interessierenden DNA-Fragments und der Menge des amplifizierten Standard-DNA-Fragments, wobei dieses Verhältnis identisch ist mit demjenigen zwischen den Mengen der markierten DNA-Fragmente, die jeweils aus den in der Stufe (3) erhaltenen amplifizierten interessierenden und Standard-DNA-Fragmenten stammen.

2. Verfahren nach Anspruch 1, dadurch gekennzeichnet, daß die Standard- und die interessierenden DNA-Fragmente sich durch mindestens eine Stelle unterscheiden, die auf irgendeine geeignete Weise reparabel ist.

3. Verfahren nach Anspruch 1 oder 2, dadurch gekennzeichnet, daß die Größen der interessierenden und der Standard-DNA-Fragmente sich um mindestens 1 und höchstens 10 % der Nucleotide oder um 1 bis 5 Nucleotide pro Strang voneinander unterscheiden.

4. Verfahren zur Bestimmung der Menge eines interessierenden DNA-Fragments durch enzymatische Amplifikation von DNA in vitro nach einem der Ansprüche 1 bis 3, dadurch gekennzeichnet, daß man in der Stufe (3) dem in der Stufe (2) erhaltenen Reaktionsmedium ein drittes markiertes Starter-Oligonucleotid zugibt, das ein gemeinsamer Starter für die interessierenden und die Standard-DNA-Fragmente ist, und daß man mit dem genannten dritten Starter einen oder mehrere zusätzliche Amplifikationscyclen so durchführt, daß im Verlaufe eines Cyclus nach der Denaturierung der DNA der genannte dritte Starter sich mit den genannten Fragmenten an einer geeigneten Stelle spezifisch hybridisiert, so daß eine Verlängerung durch DNA-Polymerase zu zwei Typen von markierten DNA-Fragmenten führt, die unterschiedliche Größen haben, je nachdem, ob sie jeweils aus interessierenden oder Standard-DNA-Fragmenten stammen, und die jeweils nur einen Teil der jeweils zuletzt genannten enthalten.

5. Verfahren nach einem der Ansprüche 1 bis 4, dadurch gekennzeichnet, daß das Standard-DNA-Fragment dem interessierenden DNA-Fragment entspricht, in dem eine oder mehrere Deletionen, Mutationen und/oder Additionen von Nucleotiden durchgeführt worden sind.

6. Verfahren nach einem der Ansprüche 4 oder 5, dadurch gekennzeichnet, daß das Standard-DNA-Fragment dem interessierenden DNA-Fragment entspricht, in dem eine Deletion, eine Mutation oder eine Addition von 1 bis 5 Nucleotiden, vorzugsweise von 3 bis 4 Nucleotiden, an einer Stelle durchgeführt worden ist und bei dem der genannte dritte Starter ein Oligonucleotid ist, das komplementär zu einer Sequenz ist, die stromaufwärts von der genannten Deletion, Mutation oder Addition angeordnet ist.

7. Verfahren zur Bestimmung der Menge eines interessierenden DNA-Fragments nach einem der Ansprüche 1 bis 6, dadurch gekennzeichnet, daß

a) die interessierenden und die Standard-DNA-Fragmente die gleiche Größe haben und sich in der Sequenz durch eine Restriktionsstelle einer speziellen Endonuclease oder durch irgendeine andere Schnittstelle, die auf irgendeine andere Weise erzielbar ist, voneinander unterscheiden,

b) man in der Stufe (3) zu dem in der Stufe (2) erhaltenen Reaktionsmedium ein drittes markiertes Starter-Oligonucleotid zugibt, das ein gemeinsamer Starter für die interessierenden und die Standard-DNA-Fragmente ist, und daß man eine oder mehrere zusätzliche Amplifikations-Cyclen mit dem genannten dritten Starter in der Weise durchführt, daß im Verlaufe eines Cyclus nach der Denaturierung der DNA der genannte dritte Starter sich spezifisch mit den genannten Fragmenten an einer geeigneten Stelle hybridisiert, so daß eine Verlängerung durch DNA-Polymerase zwei Typen von markierten DNA-Fragmenten ergibt, die sich voneinander unterscheiden, je nachdem, ob sie jeweils aus interessierenden bzw. Standard-DNA-Fragmenten stammen, und daß eines der genannten Fragmente immer die genannte Restriktionsstelle enthält, und

c) man vor der Stufe (4) eine Digestion der Amplifikations-Produkte der Stufe (3) mit Hilfe der genannten Endonuclease oder durch irgendeine andere Schneidemethode, die der Schnittstelle entspricht, in der Weise durchführt, daß Fragmente entstehen, die unterschiedliche Größen haben, je nachdem, ob sie aus interessierenden oder Standard-DNA-Fragmenten stammen.

8. Verfahren nach einem der Ansprüche 1 bis 7, dadurch gekennzeichnet, daß die Bestimmung in der Stufe (4) durchgeführt wird

- durch Trennung der markierten DNA-Fragmente, die aus amplifizierten interessierenden und Standard-DNA-Fragmenten stammen, nach ihrer Größe durch Elektrophorese auf einem Gel und
- anschließende Bestimmung der Intensitäten der Signale, die dem Marker des oder der jeweiligen genannten Starter für die markierten DNA-Fragmente entsprechen, die jeweils aus interessierenden bzw. Standard-DNA-Fragmenten stammen.

9. Verfahren nach einem der Ansprüche 1 bis 7, dadurch gekennzeichnet, daß man die markierten amplifizierten DNA-Fragmente, die aus diesen amplifizierten interessierenden und Standard-DNA-Fragmenten stammen, nach ihrer Größe trennt mit Hilfe einer Elektrophorese-Vorrichtung auf einem Gel mit einer Sequenzierung der DNA.

10. Verfahren nach einem der vorhergehenden Ansprüche zur Multiparameter-Analyse, das die Analyse (Dosierung) und quantitative Bestimmung mehrerer interessierenderDNA-Fragmente in der gleichen analysierten Probe erlaubt, dadurch gekennzeichnet, daß man

- der zu analysierenden Probe, welche die genannten interessierenden DNA-Fragmente enthält, ebensoviele Standard-DNA-Fragmente zugibt, wobei die Standard-DNA-Fragmente sich von jedem der interessierenden DNA-Fragmente unterscheiden, und
- die Standard-DNA-Fragmente und die markierten Oligonucleotide in der Stufe (3) so auswählt, daß im Verlaufe der Stufe (3) Fragmente mit unterschiedlichen Größen entstehen.

11. Verfahren zur Bestimmung der Menge eines interessierenden DNA-Fragments nach einem der Ansprüche 1 bis 5 durch enzymatische Amplifikation in vitro, dadurch gekennzeichnet, daß man in der Stufe (3) dem in der Stufe (2) erhaltenen Reaktionsmedium zwei Oligonucleotide, Sonden oder Starter zugibt, die verschieden sind von den Amplifikations-Startern der Stufe (2), die jeweils spezifisch sind für die interessierenden bzw. die Standard-DNA-Fragmente und die mit unterschiedlichen Markern markiert sind; daß man dann gegebenenfalls einen oder mehrere zusatzliche Amplifikations-Cyclen mit den genannten beiden markierten Starter-Oligonucleotiden in der Weise durchführt, daß im Verlaufe eines Cyclus nach der Denaturierung der DNA und der Verlängerung durch eine DNA-Polymerase die beiden genannten markierten Oligonucleotide zwei Typen von DNA-Fragmenten ergeben, die durch Marker markiert sind, die verschieden sind, je nachdem, ob sie aus interessierenden oder Standard-DNA-Fragmenten stammen.

12. Verfahren nach Anspruch 11, dadurch gekennzeichnet, daß

a) in der Stufe (2) die Amplifikations-Starter chemisch so modifiziert werden, daß sie an eine Feststoffphase gebunden werden können, und

b) nach der Stufe (3) die Amplifikations-Produkte an die Feststoffphase gebunden werden mittels der genannten modifizierten Starter und daß dann die Feststoffphase so gewaschen wird, daß die im Überschuß vorliegenden markierten Starter, die in der Stufe (3) nicht reagiert haben, eliminiert werden.

13. Verfahren nach Anspruch 11 oder 12, dadurch gekennzeichnet, daß dann, wenn die markierten interessierenden und Standard-DNA-Fragmente sich nur an einer einzigen Stelle unterscheiden, die beiden Starter-Oligonucleotide der Stufe (3) diese Stelle enthalten.

14. Verfahren nach einem der Ansprüche 11 bis 13, dadurch gekennzeichnet, daß die Amplifikations-Starter in der Stufe (2) biotinyliert werden und mit dem Streptavidin, das an die Feststoffphase gekuppelt ist, reagieren und sich damit verbinden können.

**15.** Verfahren nach einem der Ansprüche 1 bis 14, dadurch gekennzeichnet, daß man das Verfahren unter den gleichen Bedingungen mehrmals wiederholt, wobei man jedoch von unterschiedlichen Anfangs-Mengen der Standard-DNA-Fragmente ausgeht.

**16.** Verfahren nach einem der Ansprüche 1 bis 15, dadurch gekennzeichnet, daß der oder die genannten Starter mit einem fluoreszierenden Marker markiert werden.

**17.** Verfahren nach einem der Ansprüche 1 bis 16, dadurch gekennzeichnet, daß es sich bei dem enzymatischen Amplifikations-Verfahren um das PCR-Verfahren handelt.

**18.** Kit zur Bestimmung der Menge eines interessierenden DNA-Fragments nach einem Verfahren gemäß einem der Ansprüche 1 bis 16, dadurch gekennzeichnet, daß er umfaßt:

- ein Standard-DNA-Fragment, das sich in der Größe und/oder in der Sequenz um nicht mehr als etwa 10 %, vorzugsweise um nicht mehr als 5 Nucleotide pro Strang, von dem interessierenden DNA-Fragment unterscheidet,
- die Reagentien zur Durchführung des genannten enzymatischen Amplifikations-Verfahrens in vitro und insbesondere Starter-Oligonucleotide, und
- ein oder mehrere markierte Oligonucleotide, Sonden oder Starter, die sich spezifisch hybridisieren mit den interessierenden und den Standard-DNA-Fragmenten, gegebenenfalls an einer geeigneten Stelle, so daß eine Verlängerung durch DNA-Polymerase markierte DNA-Fragmente mit unterschiedlichen Größen und/oder Sequenzen ergibt, oder mit Markern, die verschieden sind, je nachdem, ob sie jeweils aus interessierenden bzw. Standard-DNA-Fragmenten stammen.

## Claims

**1.** Process for determining the quantity of a DNA fragment of interest in a sample to be analysed by a method of enzymatic amplification in vitro of the said fragment, characterized in that:

1) a standard DNA fragment which differs from the DNA fragment of interest but can be amplified with the same oligonucleotide primers is added to the sample to be analysed containing the DNA fragment of interest, the standard DNA fragment and the fragment of interest differing in sequence and/or in size by not more than approximately 10%, and preferably by not more than 5 nucleotides per strand,
2) the DNA fragment of interest and the standard fragment are coamplified with the same oligonucleotide primers, preferably to saturation of the amplification of the DNA fragment of interest,
3) to the reaction medium obtained in step 2), there are added:

- either two types of labelled oligonucleotide probes which are each specific for the DNA fragment of interest and the standard fragment, respectively, and different from the amplification oligonucleotide primers of step 2),
- or one or more labelled oligonucleotide primer(s), specific for the DNA fragment of interest and the standard fragment and different from the said oligonucleotide primers of step 2), and one or more additional amplification cycle(s) with the said labelled oligonucleotide primer(s) is/are performed, so that, during a cycle, after denaturation of the DNA, the said labelled oligonucleotide primer(s) hybridize(s) with the said fragments at a suitable site in order that an elongation with the DNA polymerase generates labelled DNA fragments of different sizes and/or sequences and/or with different labels according to whether they originate from the DNA fragment of interest or the standard fragment, respectively, and then

4) the initial quantity of DNA fragment of interest is determined as being the product of the initial quantity of standard DNA fragment and the ratio of the quantity of amplified DNA fragment of interest to the quantity of amplified standard DNA fragment, which ratio is identical to that of the quantities of the labelled DNA fragments originating from the amplified DNA fragment of interest and the amplified standard fragment, respectively, obtained in step 3).

**2.** Process according to Claim 1, characterized in that the standard DNA fragment and the DNA fragment of interest differ in at least one site which can be identified by some appropriate means.

3. Process according to Claim 1 or 2, characterized in that the sizes of the DNA fragment of interest and the standard fragment differ by at least 1 and by at most 10% of nucleotides or by 1 to 5 nucleotides per strand.

4. Process for determining the quantity of a DNA fragment of interest by a method of enzymatic amplification in vitro of DNA according to one of Claims 1 to 3, characterized in that, in step 3), a third labelled oligonucleotide primer is added to the reaction medium obtained in step 2), the primer being common to the DNA fragment of interest and the standard fragment, and one or more additional amplification cycle(s) is/are performed with the said third primer, so that, during a cycle, after denaturation of the DNA, the said third primer hybridizes specifically with the said fragments at a suitable site in order that an elongation with the DNA polymerase generates two types of labelled DNA fragments of different sizes according to whether they originate from the DNA fragment of interest or the standard fragment, respectively, and each containing only a portion of the latter fragments, respectively.

5. Process according to one of Claims 1 to 4, characterized in that the standard DNA fragment corresponds to the DNA fragment of interest in which one or more deletion(s), mutation(s) and/or addition(s) of nucleotides has/have been performed.

6. Process according to either of Claims 4 and 5, characterized in that the standard DNA fragment corresponds to the DNA fragment of interest in which a deletion, mutation or addition at one site of 1 to 5 nucleotides, and preferably 3 to 4, has been performed, and in that the said third primer is an oligonucleotide complementary to a sequence located upstream of the said deletion, mutation or addition.

7. Process for determining the quantity of a DNA of interest according to one of Claims 1 to 6, characterized in that:

a) the standard DNA fragment and the fragment of interest have the same size and differ in sequence by a restriction site for a particular endonuclease, or any other site of cleavage which can be produced by any other means, and
b) in step 3), a third labelled oligonucleotide primer is added to the reaction medium obtained in step 2), the primer being common to the DNA fragment of interest and the standard fragment, and one or more additional amplification cycle(s) is/are performed with the said third primer, so that, during a cycle, after denaturation of the DNA, the said third primer hybridizes specifically with the said fragments at a suitable site in order that an elongation with the DNA polymerase generates two types of labelled DNA fragments which differ according to whether they originate from the DNA fragment of interest or the standard fragment, respectively, in that one of the said fragments still contains the said restriction site, and
c) before step 4), a digestion of the amplification products of step 3) is performed, using the said endonuclease or any other means of cleavage corresponding to the cleavage site, so as to generate fragments of different sizes according to whether they originate from the DNA fragment of interest or the standard fragment.

8. Process according to one of Claims 1 to 7, characterized in that the determination of step 4) is done by

- separating the labelled DNA fragments originating from the amplified DNA fragment of interest and the amplified standard fragment according to their size by gel electrophoresis, and then by
- detecting the intensities of the signals corresponding to the label of the said respective primer(s) for the labelled DNA fragments originating from the DNA fragment of interest and the standard fragment, respectively.

9. Method according to one of Claims 1 to 7, characterized in that the labelled amplified DNA fragments originating from the amplified DNA fragment of interest and the amplified standard fragment are separated according to their size, using a device for gel electrophoresis with DNA sequencing.

10. Process according to one of the preceding claims for multiparametric analysis, enabling several DNA fragments of interest to be assayed and quantified in the same sample under analysis, characterized in that:

- to the sample to be analysed containing the said DNA fragments of interest, an equal number of standard DNA fragments are added, the standard DNA fragments being different for each of the DNA fragments of interest, and
- the standard DNAs and the labelled oligonucleotides of step 3) are chosen in such a way as to generate fragments of different sizes during step 3).

11. Process for determining the quantity of a DNA fragment of interest according to one of Claims 1 to 5, by a method of enzymatic amplification in vitro, characterized in that, in step 3), two oligonucleotide probes or primers different from the amplification primers of step 2), specific for the DNA fragment of interest and the standard fragment, respectively, and labelled with different labels, are added to reaction medium obtained in step 2); one or more additional amplification cycle(s) is/are then performed, if appropriate, with the said two labelled oligonucleotide primers, so that, during a cycle, after denaturation of the DNA and elongation with a DNA polymerase, the said two labelled oligonucleotides generate two types of DNA fragments, labelled with different labels according to whether they originate from DNA of interest or standard DNA.

12. Process according to Claim 11, characterized in that:

    a) in step 2), the amplification primers are chemically modified so that can they be linked to a solid phase, and
    b) after step 3), the amplification products are linked to the solid phase via the said modified primers, and the solid phase is then washed so as to remove the excess unreacted labelled primers of step 3).

13. Process according to Claim 11 or 12, characterized in that, if the labelled DNA fragment of interest and the labelled standard fragment differ in only one site, both oligonucleotide primers of step 3) include this site.

14. Process according to one of Claims 11 to 13, characterized in that the amplification primers of step 2) are biotinylated, and can react and can bind to streptavidin, which is coupled to the solid phase.

15. Process according to one of Claims 1 to 14, characterized in that the process is repeated several times under the same conditions but starting with different initial quantities of standard DNA fragments.

16. Process according to one of Claims 1 to 15, characterized in that the said primer(s) is/are labelled with a fluorescent label.

17. Process according to one of Claims 1 to 16, characterized in that the method of enzymatic amplification is the PCR method.

18. Kit for determining the quantity of a DNA fragment of interest by a process according to one of Claims 1 to 16, characterized in that it contains:

    - a standard DNA fragment differing in size and/or in sequence by not more than approximately 10%, and preferably by not more than 5 nucleotides per strand, from the DNA fragment of interest,
    - the reactants for carrying out the said method of enzymatic amplification in vitro, and in particular oligonucleotide primers,
    - one or more labelled oligonucleotide probe(s) or primer(s), which hybridize(s) specifically with the standard DNA fragment and the fragment of interest, as appropriate, at a suitable site in order that an elongation with the DNA polymerase generates labelled DNA fragments of different sizes and/or sequences or with different labels according to whether they originate from the DNA fragment of interest or the standard fragment, respectively.

```
H2-I                                                              CTGACCT  GGCAGTTGAA
H2-III
pH-2         ...GCCCTGG  GCTTCTACCC  TGCTGATATC  ACCCTGACCT  GGCAGTTGAA
pH-2-stand   ...GCCCTGG  GCTTCTACCC  TGCTGATATC  ACCCTGACCT  GGCAGTTGAA
H2-II

H2-I         TGG
H2-III
pH-2         TGGGGAGGAC  CTGACCCAGG  ACATGGAGCT  TGTAGAGACC  AGGCCTGCAG
pH-2-stand   TGGGGAGGAC  CTGACCCAGG  ACATGGAGCT  TGTAGAGACC  AGGCCTGCAG
H2-II

H2-I                                      XCTG  TGGTGGTGCC  TCTTGG
H2-III
pH-2         GGGATGGAAC  CTTCCAGAAG  TGGGCAGCTG  TGGTGGTGCC  TCTTGGGAAG
pH-2-stand   GGGATGGAAC  CTTCCAGAAG  TGGGCAGCTG  TGGTGGTGCC  TCTTGGGAAG
H2-II

H2-I
H2-III
pH-2         GAGCAGAATT  ACACATGCCA  TGTGCACCAT  AAGGGGCTGC  CTGAGCCTCT
pH-2-stand   GAGCAGAATT  ACACATGCCA  TGTGCACCAT  AAGGGGCTGC  CTGAGCCTCT
H2-II

H2-I
H2-III
pH-2         CACCCTGAGA  TGGA----AG  CTTCCTCCAT  CCACTGTCTC  CAACACGGTA
pH-2-stand   CACCCTGAGA  TGGAAGCTAG  CTTCCTCCAT  CCACTGTCTC  CAACACGGTA
H2-II

H2-I
H2-III
pH-2         ATCATTGCTG  TTCTGGTTGT  CCTTGGAGCT  GCAATAGTCA  CTGGAGCTGT
pH-2-stand   ATCATTGCTG  TTCTGGTTGT  CCTTGGAGCT  GCAATAGTCA  CTGGAGCTGT
H2-II                                           GGAACCTCGA  CGTTATCAGT

H2-I
H2-III
pH-2         ...
pH-2-stand   ...
H2-II
```

EP 0 613 503 B1

FIG_1

taille
(en nucléotide)    159   163        159   163        159   163

FIG_2a

FIG_2b

FIG_3

FIG_4

```
Amorce V                            CTGAATG CCCAGACAGC TCCAAGC
                                    ::::::: :::::::::: :::::::
BW5147        AGTCGTTTTA TACCTGAATG CCCAGACAGC TCCAAGCTAC TTTTACATAT
              :::::::::: :::::::::: :::::::::: :::::::::: ::::::::::
K17           AGTCGTTTTA TACCTGAATG CCCAGACAGC TCCAAGCTAC TTTTACATAT


              ATCTGCCGTG GATCCAGAAG ACTCAGCTGT CTATTTTTGT GCCAGCAGCC
              :::::::::: :::::::::: :::::::::: :::::::::: ::::::::::
              ATCTGCCGTG GATCCAGAAG ACTCAGCTGT CTATTTTTGT GCCAGCAGCC


              AGATAACTAG TAACCAAGAC ACCCAGTACT TTGGGCCAGG CACTCGGCTC
              : :  :::  :       :::: : :::::::::: ::::::::::::::::::::::
              A-ACGACTGG GGG---AGAC A-CCAGTACT TTGGGCCAGG CACTCGGCTC
                                    :::::::: :::::::::: :::
Amorce J                           CCAGTACT TTGGGCCAGG CAC


              CTCGTGTAGA GGATCTGAGA AATGTGACTC CACCCAAGG
              :::::::::: :::::::::: :::::::::: :::::::::
              CTCGTGTAGA GGATCTGAGA AATGTGACTC CACCCAAGG
                                   :::: :::::::::::: :::::::::
Amorce C                           GAGA AATGTGACTC CACCCAAG
```

# FIG.5

FIG.6

# FIG.7

```
Ald -I                                                        GACCCAC CCCGTCCTGT
                                                             ::::::: ::::::::::::
Aldolase H    AGTCCTTTCG CCTACCCACC GGCGTACCAG GCAGACCCAC CCCGTCCTGT
              :::::::::: :::::::::: :::::::::: :::::::::: ::::::::::
ADN standard  AGTCCTTTCG CCTACCCACC GGCGTACCAG GCAGACCCA  CCCGTCCTGT


              GCC
              :::
              GCCAGGAAAG CAACTGCCAC CGGCACCATG CCCCACCCAT ACCCAGCACT
              :::::::::: :::::::::: ::::::     :::::::::: ::::::::::
              GCCAGGAAAG CAACTGCCAC CGGCAC---- CCCCACCCAT ACCCAGCACT


              GACCCCGGAG CAGAAGAAGG AGCTGTCTGA CATCGCTCAC CGCATTGTGG
              :::::::::: :::::::::: :::::::::: :::::::::: ::::::::::
              GACCCCGGAG CAGAAGAAGG AGCTGTCTGA CATCGCTCAC CGCATTGTGG
                                   :: :::::::::::: ::::::::::: :
Ald - III                         GG AGCTGTCTGA CATCGCTCAC C


              CTCCGGGCAA GGGCATCCTG GCTGCAGATG AGTCCACCGG AAGCATTGCC
              :::::::::: :::::::::: :::::::::: :::::::::: ::::::::::
              CTCCGGGCAA GGGCATCCTG GCTGCAGATG AGTCCACCGG AAGCATTGCC


              AAGCGCCTGC AGTCCATTGG CACCGAGAAC ACCGAGGAGA ACAGGCGCTT
              :::::::::: :::::::::: :::::::::: :::::::::: ::::::::::
              AAGCGCCTGC AGTCCATTGG CACCGAGAAC ACCGAGGAGA ACAGGCGCTT


              CTACCGCCAG CTGCTGCTGA CTGCAGACGA CCGTGTGAAT CCCTGCATTG
              :::::::::: :::::::::: :::::::::: :::::::::: ::::::::::
              CTACCGCCAG CTGCTGCTGA CTGCAGACGA CCGTGTGAAT CCCTGCATTG


              GGGGGGTGAT CCTCTTCCAC GAGACACTGT ACCAGAAGGC AGATGATGGA
              :::::::::: :::::::::: :::::::::: :::::::::: ::::::::::
              GGGGGGTGAT CCTCTTCCAC GAGACACTGT ACCAGAAGGC AGATGATGGA
                :::::: :::::::::: :::
Ald - II        GGGTGAT CCTCTTCCAC GAG



Ubi -I                                                        GACGGGCA AGACCATCAC
                                                             :::::::: ::::::::::::
Ubiquitine    CGCGCCAACA TGCAGATCTT CGTGAAGACC CTGACGGGCA AGACCATCAC
              :::::::::: :::::::::: :::::::::: :::::::::: ::::::::::
ADN standard  CGCGCCAACA TGCAGATCTT CGTGAAGACC CTGACGGGCA AGACCATCAC


              TCTTGAGGTC GAGCCCAGTG ACACCATCGA GAATGTCAAG GCCAAGATCC
              :::::::::: :::::::::: :::::::::: :::::::::: ::::::::::
              TCTTGAGGTC GAGCCCAGTG ACACCATCGA GAATGTCAAG GCCAAGATC
              ::
              TC
```

# FIG.7 suite

```
            AAGACAAGGA AGGCATCCCA CCTGACCAGC AGAGGCTGAT ATTCGCGGGC
            :::::::::: :::::::::: :::::::::: :::::::::: ::::::::::
            AAGACAAGGA AGGCATCCCA CCTGACCAGC AGAGGCTGAT ATTCGCGGGC


Ubi - III         GG AGGATGGCCG CACCCTGTCC
                  :: :::::::::: ::::::::::
            AAACAGCTGG AGGATGGCCG CACCCTGTCC GACTACAACA TCCAGAAAGA
            :::::::::: :::::::::: :::::::::: :::::::::: ::::::::::
            AAACAGCTGG AGGATGGCCG CACCCTGTCC GACTACAACA TCCAGAAAGA


            GTCCACCTTG CACCTGGTGC TGCGTCTGCG CGGTGGCATC ATTGAGCCAT
            :::::::::: ::::::::::    :::::: :::::::::::: ::::::::::
            GTCCACCTTG CACCTGGTGC ----TCTGCG CGGTGGCATC ATTGAGCCAT


            CCCTTCGTCA GCTTGCCCAG AAGTACAACT GTGACAAGAT GA
            :::::::::: :::::::::: :::::::::: :::::::::: ::
            CCCTTCGTCA GCTTGCCCAG AAGTACAACT GTGACAAGAT GA
                       :::::::::: ::::::::::
Ubi - II              GCTTGCCCAG AAGTACAACT


P1A - I                  A ACAAGAAACC AGACAAAGCC
                         : :::::::::: ::::::::::
P1A         CCTTTGTGCC ATGTCTGATA ACAAGAAACC AGACAAAGCC CACAGTGGCT
            :::::::::: :::::::::: :::::::::: :::::::::: ::::::::::
ADN standard CCTTTGTGCC ATGTCTGATA ACAAGAAACC AGACAAAGCC CACAGTGGCT


            CAGGTGGTGA CGGTGATGGG AATAGGTGCA ATTTATTGCA CCGGTACTCC
            :::::::::: :::::::::: :::::::::: :::::::::: ::::::::::
            CAGGTGGTGA CGGTGATGGG AATA----CA ATTTATTGCA CCGGTACTCC


            CTGGAAGAAA TTCTGCCTTA TCTAGGGTGG CTGGTCTTCG CTGTTGTCAC
            :::::::::: :::::::::: :::::::::: :::::::::: ::::::::::
            CTGGAAGAAA TTCTGCCTTA TCTAGGGTGG CTGGTCTTCG CTGTTGTCAC
                                                      :: ::::::::::
P1A - III                                            CG CTGTTGTCAC


            AACAAGTTTT CTGGCGCTCC AGATGTTCAT AGACGCCCTT TATGAGGAGC
            :::::::::: :::::::::: :::::::::: :::::::::: ::::::::::
            AACAAGTTTT CTGGCGCTCC AGATGTTCAT AGACGCCCTT TATGAGGAGC
            :::::::::: :::::
            AACAAGTTTT CTGGC


            AGTATGAAAG GGATGTGGCC TGGATAGCCA GGCAAAGCAA GCGCATGTCC
            :::::::::: :::::::::: :::::::::: :::::::::: ::::::::::
            AGTATGAAAG GGATGTGGCC TGGATAGCCA GGCAAAGCAA GCGCATGTCC


            TCTGTCGATG AGGATGAAGA CGATGAGGAT GATGAGGATG ACTACTACGA
            :::::::::: :::::::::: :::::::::: :::::::::: ::::::::::
            TCTGTCGATG AGGATGAAGA CGATGAGGAT GATGAGGATG ACTACTACGA
```

# FIG_7 suite

```
CGACGAGGAC GACGACGACG ATGCCTTCTA TGATGATGAG GATGATGAGG
:::::::::: :::::::::: :::::::::: :::::::::: ::::::::::
CGACGAGGAC GACGACGACG ATGCCTTCTA TGATGATGAG GATGATGAGG


AAGAAGAATT GGAGAACCTG ATGGATGATG AATCAGAAGA TGAGGCCGAA
:::::::::: :::::::::: :::::::::: :::::::::: ::::::::::
AAGAAGAATT GGAGAACCTG ATGGATGATG AATCAGAAGA TGAGGCCGAA


GAAGAGATGA GCGTGGAAAT GGGTGCCGGA GCTGAGGAAA TGGGTGCTGG
:::::::::: :::::::::: :::::::::: :::::::::: ::::::::::
GAAGAGATGA GCGTGGAAAT GGGTGCCGGA GCTGAGGAAA TGGGTGCTGG
                           .                     ::::::::
                                                GGTGCTGG
```

P1A - II

```
CGCTAACTGT GCCTGTGTTC CTGGCCATCA TTTAAGGAAG AATGAAGTGA
:::::::::: :::::::::: :::::::::: :::::::::: ::::::::::
CGCTAACTGT GCCTGTGTTC CTGGCCATCA TTTAAGGAAG AATGAAGTGA
:::::::::: :
CGCTAACTGT G
```

FIG.8

| taille : (nucléotide) | 159 | 163 | 159 | 163 |
|---|---|---|---|---|
| nombre de copies de pH2* | 3000 | | 70000 | |
| surface de pic | 11204 | 2379 | 5989 | 19018 |

FIG.9a

EP 0 613 503 B1

FIG_9b

FIG_10

A.

B.

TAILLE EN NUCLEOTIDES

SURFACE ETALON/SURFACE ADNc

NOMBRE DE COPIES D'ETALON SOUMIS A L'AMPLIFICATION

□ CD8

◆ CD4

EP 0 613 503 B1

46

FIG_11